# EUROPEAN PATENT APPLICATION

(11) **EP 4 582 419 A1**
(43) Date of publication of application: **09.07.2025**
(21) Application number: 23860377.3
(22) Date of filing: 29.08.2023
(51) Int. Cl.: C07D 401/14, A61K 31/498, A61P 35/00, C07B 53/00, C07B 61/00

(54) **METHOD FOR PRODUCING QUINOXALINE DERIVATIVE**

(30) Priority: 30.08.2022 JP 2022136976
(71) Applicant: Kyowa Kirin Co., Ltd., Tokyo 100-0004 (JP)
(72) Inventor: TAKEMURA, Kazunobu, Tokyo 100-0004 (JP); NITTA, Mariko, Tokyo 100-0004 (JP); MATSUMURA, Tsutomu, Tokyo 100-0004 (JP); FUKUDA, Yuichi, Tokyo 100-0004 (JP); YAMAMOTO, Takuya, Tokyo 100-0004 (JP); HAGIHARA, Koji, Tokyo 100-0004 (JP)
(74) Representative: Vossius & Partner Patentanwälte Rechtsanwälte mbB
(86) International application number: PCT/JP2023/031342
(87) International publication number: WO 2024/048615

(57) **Abstract**

The present invention provides a method for producing a compound represented by the formula (I). The method includes a step of reacting a compound represented by the formula (II) and a compound represented by the formula (III).

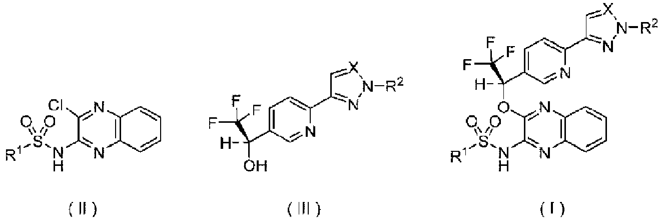

## Description

### TECHNICAL FIELD

The present invention relates to a method for producing a quinoxaline derivative, a crystal of the quinoxaline derivative, and the like.

### BACKGROUND ART

It is known that quinoxaline derivatives represented by the following formula (I) or pharmaceutical acceptable salts thereof have an inhibitory effect on the generation of kynurenine and is useful for, for example, cancer treatment. More specifically, compounds represented by the following formulas (IA) and (IB) are known (Patent Literature 1).

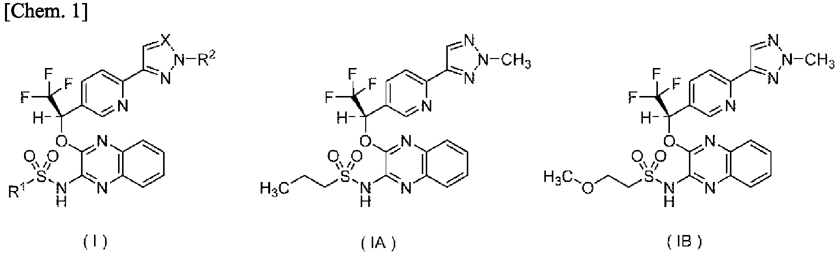

In the formula, R¹ represents lower alkyl which may be substituted with lower alkoxy or cycloalkyl, R² represents lower alkyl, and X represents N or CH.

It is also known that the generation of kynurenine is inhibited by indoleamine 2,3-dihydrogenase (IDO) inhibitors (for example, Non-Patent Literature 1).

As a method for producing the compounds represented by, for example, the formula (IA) among these quinoxaline derivatives, the following production method has been known (Patent Literature 1).

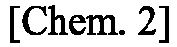

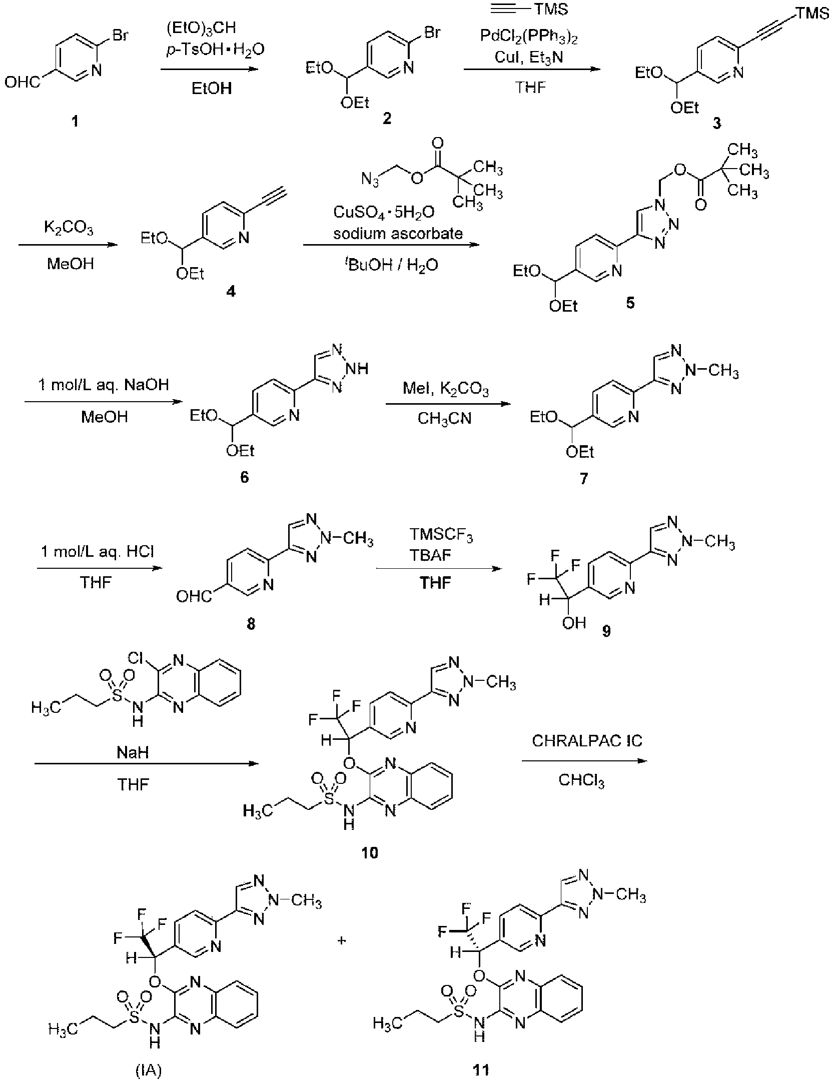

### CITATION LIST

### PATENT LITERATURE

Patent Literature 1: WO2011/142316

### NON-PATENT LITERATURE

Non Patent Literature 1: Journal of Clinical Investigation (J Clin Invest.), Vol. 117, No. 5, 1147-1154 (2007)

### SUMMARY OF INVENTION

### TECHNICAL PROBLEM

An object of the present invention is to provide an industrial method for producing a compound represented by the formula (I). Another object of the present invention is to provide a crystal of a compound represented by the formula (IA).

### SOLUTION TO PROBLEM

The present invention relates to the following (1) to (16).
(1) A method for producing a compound represented by the formula (I), the method comprising: a step of reacting a compound represented by the formula (II) and a compound represented by the formula (III), 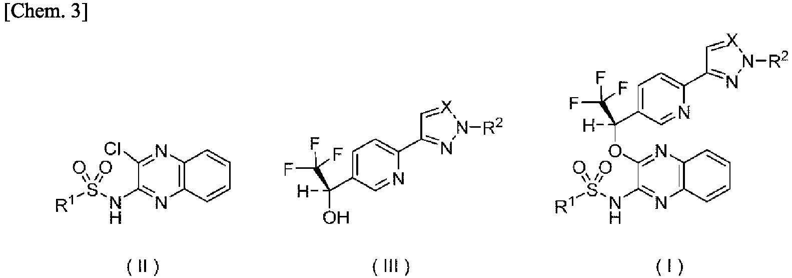 wherein R¹ represents lower alkyl which may be substituted with lower alkoxy or cycloalkyl, R² represents lower alkyl, and X represents N or CH.
(2) The method according to (1), further comprising:
   a step of obtaining the compound represented by the formula (III) from a compound represented by the formula (VI),
   wherein the step is achieved by the following two reactions:
      an asymmetric reduction reaction of a trifluoroacetyl group; and
      a condensation reaction using a compound represented by the formula (IV), 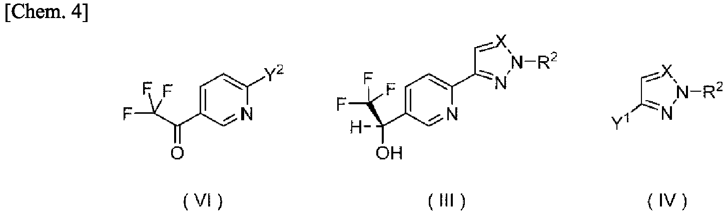
      wherein R² represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other and represent H or lower alkyl, or form a ring structure together with boron.
(3) The method according to (2), further comprising:
   a step of subjecting the compound represented by the formula (VI) to asymmetric reduction to obtain a compound represented by the formula (V); and
   a step of reacting the compound represented by the formula (V) and the compound represented by the formula (IV) to obtain the compound represented by the formula (III), 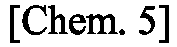 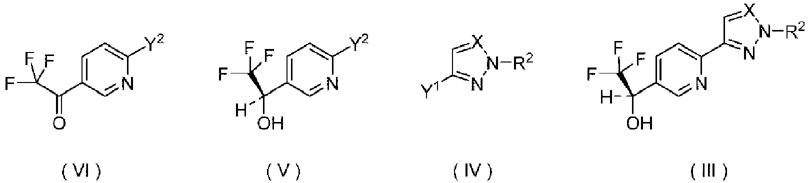
   wherein R² represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other and represent H or lower alkyl, or form a ring structure together with boron.
(3A) The method according to (1), further comprising:
   a step of subjecting the compound represented by the formula (VI) to asymmetric reduction to obtain a compound represented by the formula (V); and
   a step of reacting the compound represented by the formula (V) and the compound represented by the formula (IV) to obtain a compound represented by the formula (III), 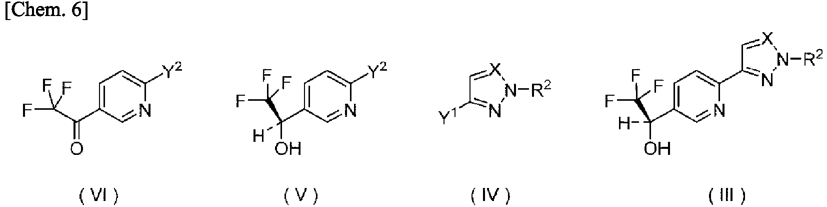
   wherein R² represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other and represent H or lower alkyl, or form a ring structure together with boron.
(4) The method according to (2), further comprising:
   a step of reacting the compound represented by the formula (VI) and the compound represented by the formula (IV) to obtain a compound represented by the formula (VIII); and
   a step of subjecting the compound represented by the formula (VIII) to asymmetric reduction to obtain the compound represented by the formula (III), 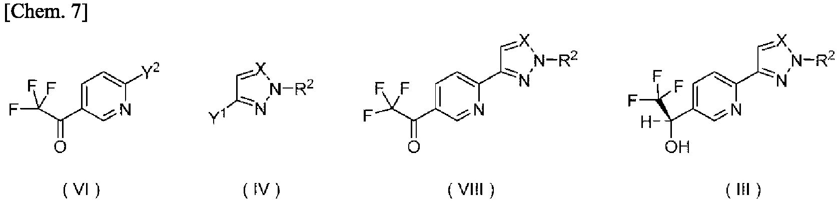
   wherein R² represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other and represent H or lower alkyl, or form a ring structure together with boron.
(4A) The method according to (1), further comprising:
   a step of reacting the compound represented by the formula (VI) and the compound represented by the formula (IV) to obtain the compound represented by the formula (VIII); and
   a step of subjecting the compound represented by the formula (VIII) to asymmetric reduction to obtain the compound represented by the formula (III), 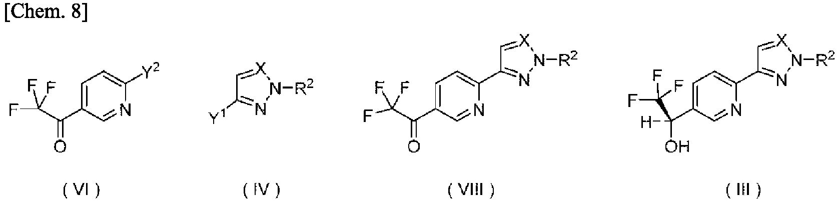
   wherein R² represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other and represent H or lower alkyl, or form a ring structure together with boron.
(5) The method according to (1), further comprising:
   a step of obtaining the compound represented by the formula (III) from a compound represented by the formula (VII),
   wherein the step is achieved by the following three reactions:
      a reaction of generating a trifluoroacetyl group from a carboxy ester group using a trifluoromethylating agent;
      an asymmetric reduction reaction of a trifluoroacetyl group; and
      a condensation reaction using the compound represented by the formula (IV), 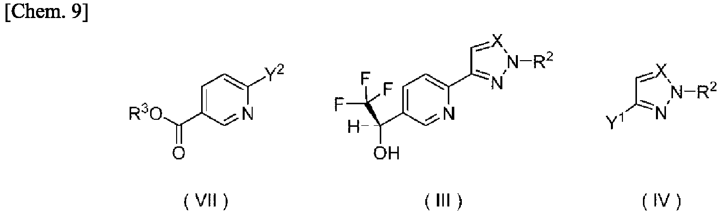
      wherein R² represents lower alkyl, R³ represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other, and represent H or lower alkyl, or form a ring structure together with boron.
(6) The method according to any one of (2) to (4), further comprising:
   a step of reacting the compound represented by the formula (VII) and a trifluoromethylating agent to obtain the compound represented by the formula (VI), 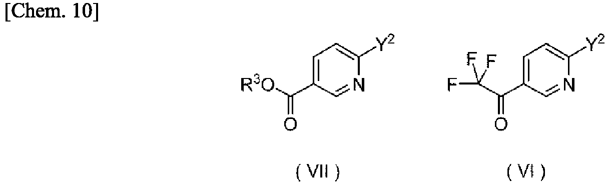
   wherein R³ represents lower alkyl, and Y² represents halogen.
      Here, any one of (2) to (4) means any one of (2), (3), (4), (3A), and (4A). Hereinafter, in this paragraph, each item number (n) includes an alphabetical item number. That is, for example, (5) also includes (5A) to (5E).
(7) The method according to (1) further comprising:
   a step of reacting the compound represented by the formula (VII) and a compound represented by the formula (IV) to obtain a compound represented by the formula (IX);
   a step of reacting the compound represented by the formula (IX) and a trifluoromethylating agent to obtain the compound represented by the formula (VIII); and
   a step of subjecting the compound represented by the formula (VIII) to asymmetric reduction to obtain the compound represented by the formula (III), 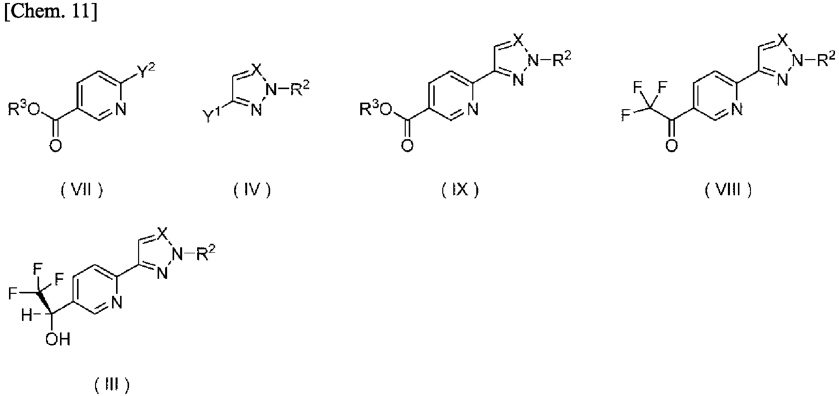
   wherein R² represents lower alkyl, R³ represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other, and represent H or lower alkyl, or form a ring structure together with boron.
(5A) The method according to (5), further comprising:
   a step of reacting the compound represented by the formula (VII) and the trifluoromethylating agent to obtain a compound represented by the formula (VI);
   a step of subjecting the compound represented by formula (VI) to asymmetric reduction to obtain the compound represented by the formula (V); and
   a step of reacting the compound represented by the formula (V) and the compound represented by the formula (IV) to obtain the compound represented by the formula (III), 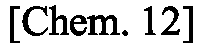 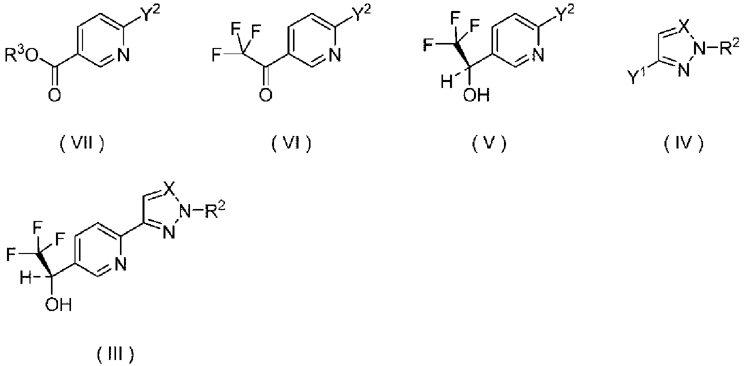
   wherein R² represents lower alkyl, R³ represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other, and represent H or lower alkyl, or form a ring structure together with boron.
(5B) The method according to (5), further comprising:
   a step of reacting the compound represented by the formula (VII) and the trifluoromethylating agent to obtain the compound represented by the formula (VI);
   a step of reacting the compound represented by the formula (VI) and the compound represented by the formula (IV) to obtain the compound represented by the formula (VIII); and
   a step of subjecting the compound represented by the formula (VIII) to asymmetric reduction to obtain the compound represented by the formula (III), 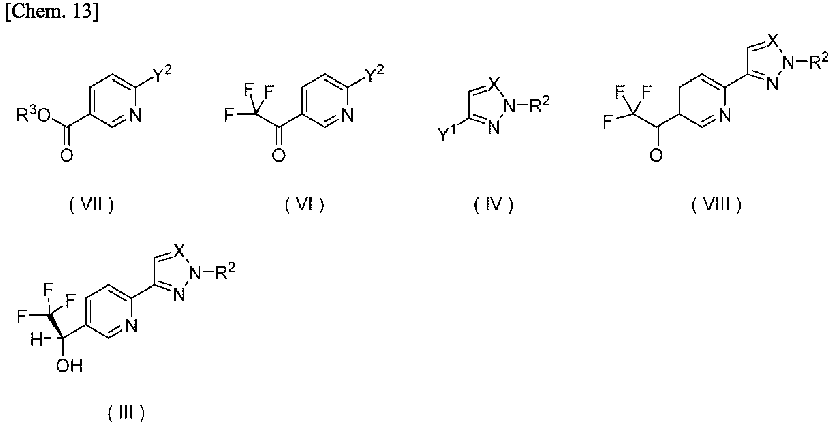
   wherein R² represents lower alkyl, R³ represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other, and represent H or lower alkyl, or form a ring structure together with boron.
(5C) The method according to (5), further comprising:
   a step of reacting the compound represented by the formula (VII) and the compound represented by the formula (IV) to obtain the compound represented by the formula (IX);
   a step of reacting the compound represented by the formula (IX) and the trifluoromethylating agent to obtain the compound represented by the formula (VIII); and
   a step of subjecting the compound represented by the formula (VIII) to asymmetric reduction to obtain the compound represented by the formula (III), 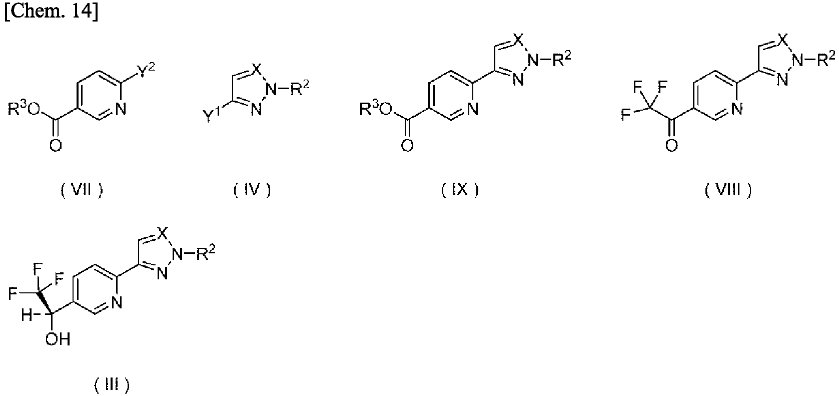
   wherein R² represents lower alkyl, R³ represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other, and represent H or lower alkyl, or form a ring structure together with boron.
(5D) The method according to (1), further comprising:
   a step of reacting the compound represented by the formula (VII) and a trifluoromethylating agent to obtain the compound represented by the formula (VI);
   a step of subjecting the compound represented by formula (VI) to asymmetric reduction to obtain the compound represented by the formula (V); and
   a step of reacting the compound represented by the formula (V) and a compound represented by the formula (IV) to obtain the compound represented by the formula (III), 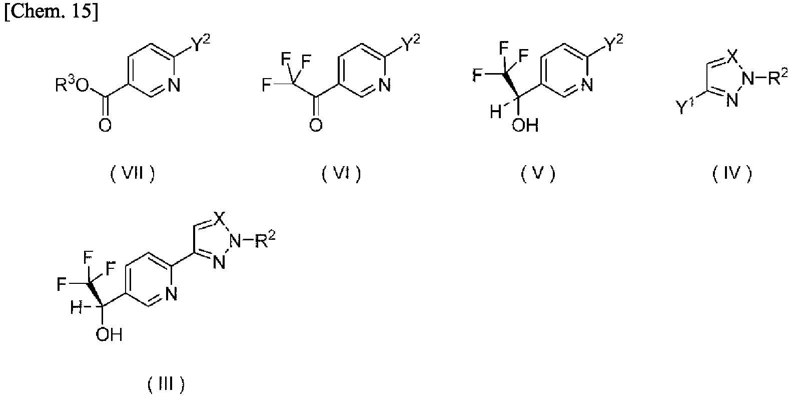
   wherein R² represents lower alkyl, R³ represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other, and represent H or lower alkyl, or form a ring structure together with boron.
(5E) The method according to (1), further comprising:
   a step of reacting the compound represented by the formula (VII) and a trifluoromethylating agent to obtain the compound represented by the formula (VI);
   a step of reacting the compound represented by the formula (VI) and the compound represented by the formula (IV) to obtain the compound represented by the formula (VIII); and
   a step of subjecting the compound represented by the formula (VIII) to asymmetric reduction to obtain the compound represented by the formula (III), 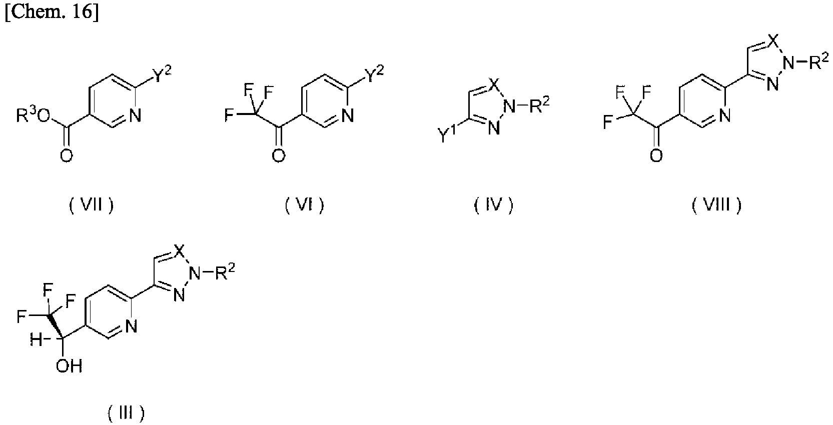
   wherein R² represents lower alkyl, R³ represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other, and represent H or lower alkyl, or form a ring structure together with boron.
(8) The method according to (1), further comprising:
   a step of reacting a compound represented by the formula (V) and a compound represented by the formula (IV) to obtain the compound represented by the formula (III), 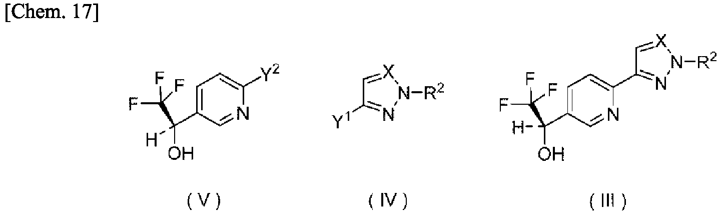
   wherein R² represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other and represent H or lower alkyl, or form a ring structure together with boron.
(9) The method according to (1), further comprising:
   a step of subjecting the compound represented by the formula (VIII) to asymmetric reduction to obtain the compound represented by the formula (III), 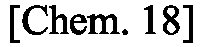 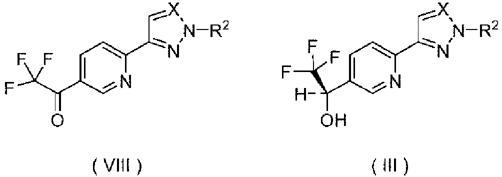
   wherein R² represents lower alkyl, and X represents N or CH.
(10) The method according to any one of (2) to (7) and (9), wherein an asymmetric reduction catalyst represented by the formula (M) is used in the asymmetric reduction reaction, 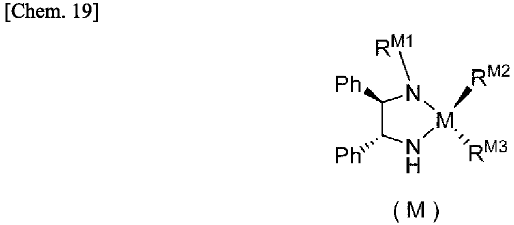 wherein M represents a metal species selected from ruthenium, rhodium, and iridium, R^{M1} represents p-toluene sulfonyl, methane sulfonyl, or benzyl sulfonyl, R^{M2} represents mesitylene, cumene, or pentamethylcyclopentadienyl, and R^{M3} is not present or represents chloro- or trifluoromethanesulfonate.
(11) The method according to (10), wherein the asymmetric reduction catalyst represented by the formula (M) is formed in a reaction of (pentamethylcyclopentadienyl)iridium (III) dichloride (dimer) and (R,R)-N-(2-amino-1,2-diphenylethyl)-p-toluenesulfonamide.
(12) The method according to any one of (1) to (11), wherein R¹ represents propyl, R² represents methyl, and X represents N.
(13) The method according to (12), further comprising: a step of recrystallizing the compound represented by the formula (I) using an alcohol-based solvent and water, 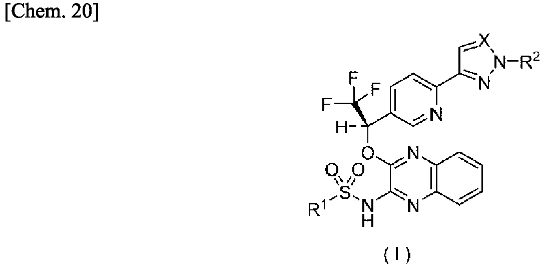 wherein R¹ represents lower alkyl which may be substituted with lower alkoxy or cycloalkyl, R² represents lower alkyl, and X represents N or CH.
(14) A crystal of a compound represented by the formula (IA), having peaks at diffraction angles (2θ ± 0.2°) of 23.1°, 24.4°, and 25.6° in powder X-ray diffraction. 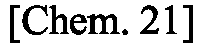 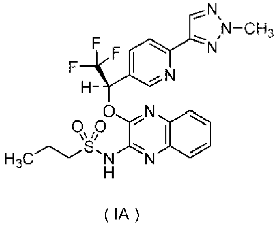
(15) The crystal according to (14), having peaks at diffraction angles (2θ ± 0.2°) of 17.0°, 21.0°, 23.1°, 24.4°, and 25.6° in the powder X-ray diffraction.
(16) The crystal according to (14), having peaks at diffraction angles (2θ ± 0.2°) of 10.5°, 12.0°, 14.3°, 15.8°, 16.3°, 17.0°, 18.0°, 18.9°, 20.2°, 21.0°, 22.4°, 22.8°, 23.1°, 24.4°, and 25.6° in the powder X-ray diffraction.
(17) A pharmaceutical preparation comprising the crystal according to any one of (14) to (16).
(18) An indoleamine 2,3-dihydrogenase inhibitor comprising the crystal according to any one of (14) to (16).
(19) The crystal according to any one of (14) to (16) for use in inhibition of indoleamine 2,3-dihydrogenase.
(20) A method for treating a disease associated with kynurenine generation and/or indoleamine 2,3-dihydrogenase expression by administering the crystal according to any one of (14) to (16) to a patient.
(21) A use of the crystal according to any one of (14) to (16) in production of the indoleamine 2,3-dihydrogenase inhibitor.

### ADVANTAGEOUS EFFECTS OF INVENTION

The present invention provides a method for producing a compound represented by the formula (I), a crystal of a compound represented by the formula (IA) and the like. The production method according to the present invention is useful as an industrial production method for an active pharmaceutical ingredient. Further, the crystal of the compound represented by the formula (IA) of the present invention is useful as the active pharmaceutical ingredient.

### BRIEF DESCRIPTION OF DRAWINGS

[FIG. 1] FIG. 1 shows a powder X-ray diffraction pattern of a type II crystal of a compound IA. The vertical axis represents a diffraction intensity (Counts/sec), and the horizontal axis represents a diffraction angle (2θ°).
[FIG. 2] FIG. 2 shows a powder X-ray diffraction pattern of a type III crystal of the compound IA. The vertical axis represents a diffraction intensity (Counts/sec), and the horizontal axis represents a diffraction angle (2θ°).

### DESCRIPTION OF EMBODIMENTS

In the present specification, a compound represented by the general formula (I) is also referred to as a compound I. The same applies to compounds denoted by other formula numbers.

In the definitions of the groups in the general formulas (I), (II), (III), (IV), (V), (VI), (VII), (VIII) and (IX), examples of a lower alkyl and a lower alkyl moiety of a lower alkoxy include linear or branched alkyls having 1 to 10 carbon atoms, and more specific examples thereof include methyl, ethyl, propyl, isopropyl, butyl, isobutyl, sec-butyl, tert-butyl, pentyl, isopentyl, neopentyl, hexyl, heptyl, octyl, nonyl, and decyl.

Examples of the cycloalkyl include cycloalkyls having 3 to 10 carbon atoms, and specific examples thereof include cyclopropyl, cyclobutyl, cyclopentyl, cyclohexyl, cycloheptyl, cyclooctyl, cyclononyl, and cyclodecanyl.

The lower alkyl which may be substituted with lower alkoxy or cycloalkyl means lower alkyl which may be substituted with lower alkoxy or lower alkyl which may be substituted with cycloalkyl, and examples thereof include methyl, ethyl, propyl, isopropyl, butyl, isobutyl, sec-butyl, tert-butyl, pentyl, isopentyl, neopentyl, hexyl, heptyl, octyl, nonyl, decyl, methoxymethyl, methoxyethyl, methoxypropyl, ethoxymethyl, ethoxyethyl, cyclopropylmethyl, cyclobutylmethyl, cyclopentylmethyl, and cyclohexylmethyl.

Here, examples of lower alkyl substituted by lower alkoxy or cycloalkyl include methoxymethyl, methoxyethyl, methoxypropyl, ethoxymethyl, ethoxyethyl, cyclopropylmethyl, cyclobutylmethyl, cyclopentylmethyl, and cyclohexylmethyl.

Halogen means a fluorine atom, a chlorine atom, a bromine atom, or an iodine atom.

Next, a method (the following scheme) for producing a compound I will be specifically described, 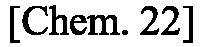 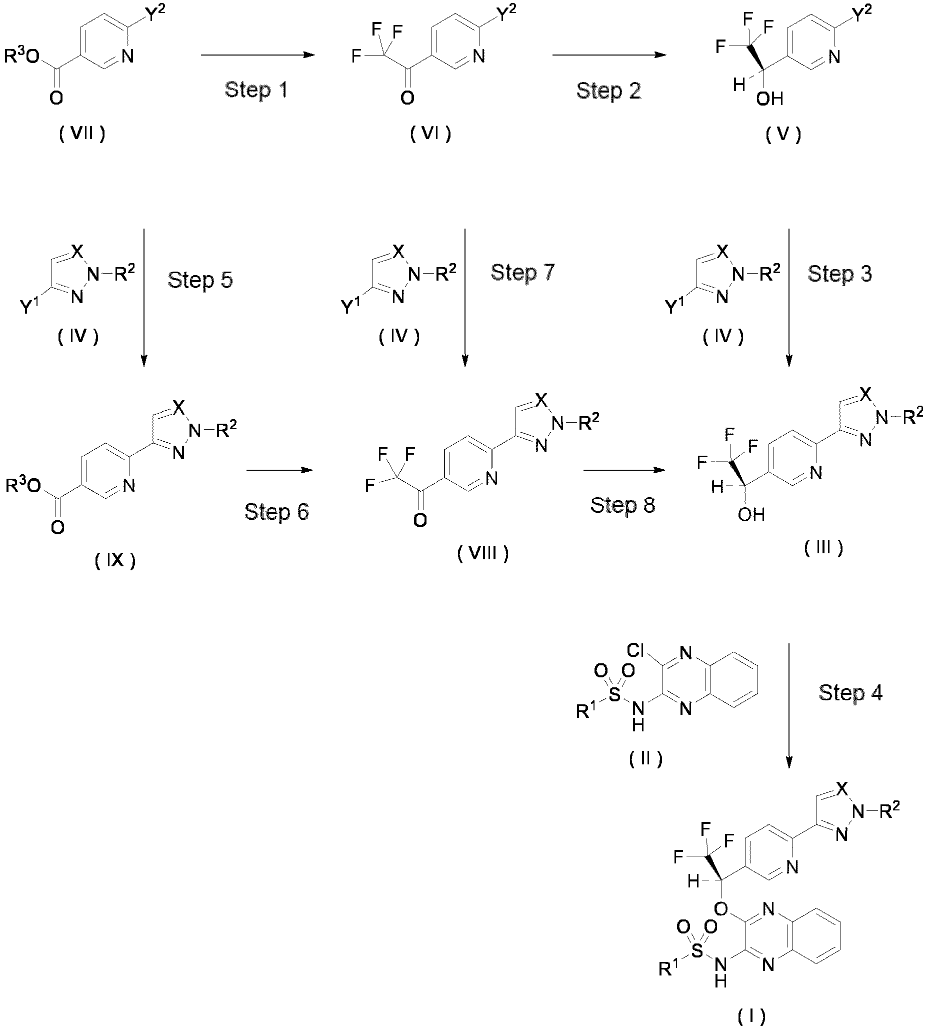 wherein R¹ represents lower alkyl which may be substituted with lower alkoxy or cycloalkyl, R² represents lower alkyl, R³ represents lower alkyl, X represents N or CH, Y¹ represents halogen, M'Y³ or B(OR⁴)₂, Y² represents halogen, Y³ represents halogen, M' represents a metal species selected from zinc or magnesium, R⁴s are the same as or different from each other and represent H or lower alkyl, or form a ring structure together with boron.

In the present invention, in the method for producing the compound I, the compounds II to IX may be used in the form of a salt, and the compound I may also be produced in the form of a salt.

The method for producing the compound I according to the present invention includes at least step 4 of reacting the compound II and the compound III.

The method for producing the compound I according to the present invention may further include step 3 or step 8 in addition to step 4, and may further include step 2 and step 3, step 7 and step 8, or step 6 and step 8 in addition to step 4.

The method for producing the compound I according to the present invention may further include a step of obtaining the compound III from the compound VI in addition to step 4. The step of obtaining the compound III from the compound VI is achieved by two reactions, an asymmetric reduction reaction of a trifluoroacetyl group and a condensation reaction using the compound IV.

The two reactions as the step of obtaining the compound III from the compound VI may be step 2 and step 3 or step 7 and step 8.

In the method for producing the compound I according to the present invention, step 2, step 3, and step 4 may be sequentially achieved, or step 7, step 8, and step 4 may be sequentially achieved.

The method for producing the compound I according to the present invention may further include, in addition to step 4, a step of obtaining the compound III from the compound VI, and may further include step 1.

In the method for producing the compound I according to the present invention, step 1, step 2, step 3, and step 4 may be sequentially achieved, or step 1, step 7, step 8, and step 4 may be sequentially achieved.

The method for producing the compound I according to the present invention may further include a step of obtaining the compound III from the compound VII in addition to step 4. The step of obtaining the compound III from the compound VII is achieved by three reactions: a reaction of generating a trifluoroacetyl group from a carboxy ester group by using a trifluoromethylating agent; an asymmetric reduction reaction of the trifluoroacetyl group; and a condensation reaction using the compound IV.

The three reactions as the step of obtaining the compound III from the compound VII may be step 1, step 2 and step 3, step 1, step 7 and step 8, or step 5, step 6 and step 8.

In the method for producing the compound I according to the present invention, step 1, step 2, step 3, and step 4 may be sequentially achieved, step 1, step 7, step 8, and step 4 may be sequentially achieved, or step 5, step 6, step 8, and step 4 may be sequentially achieved.

In the method for producing the compound I according to the present invention, any methods including at least step 4 may include any steps, and a method including the step 3 is preferred. When the method includes step 3, the method for producing the compound I according to the present invention includes step 3 and step 4, may include step 2, step 3, and step 4, and may include step 1, step 2, step 3, and step 4.

### Step 1

The compound VI can be obtained by reacting the compound VII and a trifluoromethylating agent in an appropriate solvent in the presence of a carboxylate or a fluoride salt. That is, the reaction in this step 1 is a reaction for generating a trifluoroacetyl group from a carboxy ester group in the compound VII by using a trifluoromethylating agent.

An amount of each reagent to be used is not particularly limited. For example, an amount of the trifluoromethylating agent is preferably 1.0 to 3.0 equivalent, and more preferably 1.5 to 2.0 equivalent, and an amount of the carboxylate or the fluoride salt is preferably 0.01 to 0.20 equivalent, and more preferably 0.05 to 0.10 equivalent, with respect to the amount of the compound VII.

The compound VII may be used as a commercially available product.

The trifluoromethylating agent is not particularly limited, and examples thereof include (trifluoromethyl)trimethylsilane, (trifluoromethyl)triethylsilane, and potassium trimethoxy(trifluoromethyl)boronate.

The carboxylate is not particularly limited, and examples thereof include cesium acetate, sodium acetate, potassium acetate, tetrabutylammonium acetate, cesium propionate, sodium propionate, potassium propionate, cesium pivalate, sodium pivalate, potassium pivalate, cesium octanoate, sodium octanoate, potassium octanoate, cesium 2-ethylhexanate, sodium 2-ethylhexanoate, and potassium 2-ethylhexanoate. Cesium 2-ethylhexanoate is preferred.

The carboxylate can be made present in a reaction system by adding both an appropriate carboxylic acid and an appropriate base to the reaction system. For example, in the case of cesium 2-ethylhexanoate, 2-ethylhexanoic acid and cesium carbonate are preferably used.

The fluoride salt is not particularly limited, and examples thereof include cesium fluoride, sodium fluoride, potassium hydrogen fluoride, tetrabutylammonium fluoride, and tetrabutylammonium difluorotriphenylsilicate. Cesium fluoride is preferred.

As the carboxylate or the fluoride salt, it is preferable to use a carboxylate from the viewpoint of solubility.

The solvent is not particularly limited, and examples thereof include tetrahydrofuran (THF), 2-methyltetrahydrofuran (2-MeTHF), dioxane, methyl-tert-butyl ether, dimethylformamide, dimethylacetamide, and toluene. These may be used alone or in combination. 2-MeTHF is preferred.

The amount of the solvent used is not particularly limited, and is, for example, 10 to 20 volume/weight (v/w) with respect to the compound VII.

The reaction is performed preferably at a temperature between 0°C and 30°C and more preferably at a temperature between 0°C and 15°C, generally for 30 minutes to 2 hours.

The obtained compound VI may be used in the next step without being isolated.

### Step 2

The compound V can be obtained by treating the compound VI with a metal catalyst and, if necessary, a ligand in an appropriate solvent in the presence of a base and a hydrogen source. That is, the reaction in this step 2 is an asymmetric reduction reaction for the trifluoroacetyl group in the compound VI.

An amount of each reagent used is not particularly limited. For example, with respect to the compound VI, the base is preferably 1.0 to 3.0 equivalent, and more preferably 1.5 to 2.0 equivalent, the hydrogen source is preferably 1.0 to 5.0 equivalent, and more preferably 1.0 to 1.5 equivalent, the metal catalyst is preferably 0.01 to 0.20 equivalent, and more preferably 0.02 to 0.10 equivalent, and the ligand is preferably 0.01 to 0.20 equivalent, and more preferably 0.02 to 0.10 equivalent.

The base is not particularly limited, and examples thereof include triethylamine, diisopropylethylamine, tributylamine, diazabicycloundecene, and diazabicyclononene. Triethylamine is preferred.

The hydrogen source is not particularly limited, and examples thereof include formic acid, sodium formate, hydrogen, and isopropanol. Formic acid, sodium formate, and hydrogen are preferred, and formic acid is more preferred.

The metal catalyst is not particularly limited as long as it can be used for asymmetric reduction. For example, a compound represented by the following formula (M) can be exemplified, 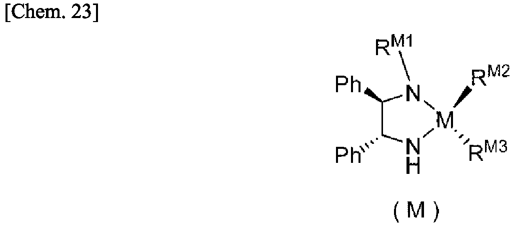 wherein M represents a metal species such as ruthenium, rhodium, or iridium, R^{M1} represents a protective group such as p-toluenesulfonyl, methanesulfonyl, or benzylsulfonyl, R^{M2} represents mesitylene, cumene, pentamethylcyclopentadienyl, or the like, and R^{M3} represents chloro, trifluoromethanesulfonate, or the like, and may not be present.

More specifically, examples of the metal catalyst include [N-[(1R,2R)-2-(amino-κN)-1,2-diphenylethyl]-4-methylbenzenesulfonamide-κN]chloro[(1,2,3,4,5-η)-1,2,3,4,5-pentamethyl-2,4-cyclopentadiene-1-yl]iridium, [N-[(1R,2R)-2-(amino-κN)-1,2-diphenylethyl]-4-methylbenzenesulfonamide-κN]chloro[(1,2,3,4,5-η)-1,2,3,4,5-pentamethyl-2,4-cyclopentadiene-1-yl]rhodium, [(R,R)-N-(2-amino-1,2-diphenylethyl)-p-toluenesulfonamide]chloro(mesitylene)ruthenium(II), [(R,R)-N-(2-amino-1,2-diphenylethyl)-p-toluenesulfonamide]chloro(cumene)ruthenium(II), [(R,R)-N-(2-amino-1,2-diphenylethyl)-p-toluenesulfonamide]triflate(mesitylene)ruthenium(II), [(R,R)-N-(2-amino-1,2-diphenylethyl)-p-toluenesulfonamide](mesitylene)ruthenium(II). Preferred is [N-[(1R,2R)-2-(amino-κN)-1,2-diphenylethyl]-4-methylbenzenesulfonamide-κN]chloro[(1,2,3,4,5-η)-1,2,3,4,5-pentamethyl-2,4-cyclopentadiene-1-yl]iridium. It is to be noted that [N-[(1R,2R)-2-(amino-κN)-1,2-diphenylethyl]-4-methylbenzenesulfonamide-κN]chloro[(1,2,3,4,5-η)-1,2,3,4,5-pentamethyl-2,4-cyclopentadiene-1-yl]iridium or [N-[(1R,2R)-2-(amino-κN)-1,2-diphenylethyl]-4-methylbenzenesulfonamide-κN]chloro[(1,2,3,4,5-η)-1,2,3,4,5-pentamethyl-2,4-cyclopentadiene-1-yl]rhodium can be generated in a reaction system by reacting (pentamethylcyclopentadienyl)iridium (III) dichloride (dimer) or (pentamethylcyclopentadienyl)rhodium(III) dichloride (dimer) with (R,R)-N-(2-amino-1,2-diphenylethyl)-p-toluenesulfonamide.

Examples of the ligand include (R,R)-N-(2-amino-1,2-diphenylethyl)-p-toluenesulfonamide, (R,R)-N-(2-amino-1,2-diphenylethyl)-p-toluenesulfonamide, (R,R)-N-(2-amino-1,2-diphenylethyl)-methanesulfonamide, and (R,R)-N-(2-amino-1,2-diphenylethyl)-benzylsulfonamide. (R,R)-N-(2-amino-1,2-diphenylethyl)-p-toluenesulfonamide is preferred.

The solvent is not particularly limited, and examples thereof include THF, 2-MeTHF, 2-propanol, ethanol, methanol, water, dichloroethane, chloroform, acetonitrile, methyl ethyl ketone, chlorobenzene, and trifluoromethylbenzene. These can be used alone or in combination. 2-MeTHF is preferred.

The amount of the solvent used is not particularly limited, and is, for example, 10 to 20 volume/weight (v/w) with respect to the compound VII.

The reaction is performed preferably at a temperature between 20°C and 60°C and more preferably at a temperature between 20°C and 40°C, generally for 3 hours to 10 hours.

The metal species derived from a metal catalyst such as a ruthenium catalyst, a rhodium catalyst, and an iridium catalyst can be removed and decolored by using activated carbon in the post-treatment after the reaction.

Optical purity of the obtained compound V can be improved by recrystallization using an appropriate solvent. In the recrystallization step of the compound V, a free form of the compound V may be used, or a salt of the compound V may be used.

The solvent used in the recrystallization step of the compound V is not particularly limited, and examples thereof include toluene, heptane, ethyl acetate, propyl acetate, butyl acetate, methyl-tert-butyl ether, and THF. These may be used alone or in combination. Toluene and heptane are preferably used, and it is preferable to perform recrystallization by utilizing the difference in solubility.

The amount of the solvent used is not particularly limited, and for example, the solvent is used in an amount of 10 to 25 volume/weight (v/w) with respect to the compound VII.

The compound V can also be obtained by treating the compound VI with an enzyme kit for chiral alcohol synthesis. The asymmetric reduction method using such an enzyme is preferable from the viewpoint of obtaining a compound having high optical purity.

The enzyme kit for chiral alcohol synthesis is not particularly limited, and for example, a commercially available product such as Chiralscreen (registered trademark) OH screening kit sold by Daicel Corporation can be used.

The compound V may be obtained in the form of a salt such as a hydrochloride in some cases.

### Step 3

The compound III can be obtained by a condensation reaction of the compound IV and the compound V in an appropriate solvent. Hereinafter, in the description of step 3, in the compound IV, R² represents lower alkyl, X represents N or CH, Y¹ represents any one of halogen, M'Y³, or B(OR⁴)₂, Y³ represents halogen, M' represents a metal species selected from zinc and magnesium, and R⁴s are the same as or different from each other and represent H or lower alkyl, or form a ring structure together with boron.

Y¹ in the compound IV is any one of halogen, M'Y³, or B(OR⁴)₂, and when Y¹ represents halogen, the compound V and the compound IV wherein Y¹ is M'Y³ or B(OR⁴)₂ which is obtained from the compound IV wherein Y¹ is halogen, may be subjected to a condensation reaction.

A method of introducing M'Y³ or B(OR⁴)₂ into Y¹ in the compound IV (Y¹ represents halogen) is not particularly limited. For example, (i) M'Y³ (M' represents magnesium. That is, Y¹ represents MgY³) can be introduced into Y¹ in the compound IV by reacting the compound IV with a Grignard reagent, and next, (ii) M'Y³ (M' represents zinc. That is, Y¹ represents ZnY³.) or B(OR⁴)₂ can be introduced into Y¹ in the compound IV by reacting the obtained reaction mixture in (i) with zinc halide, organodiboron or boronic acid ester. The compound III can be obtained by (iii) further subjecting the compound V to a condensation reaction with the reaction mixture obtained in (i) or (ii) in the presence of a metal catalyst.

When Y¹ represents MgY³, the compound III can be obtained by (i) reacting the compound IV with zinc halide, organodiboron or boronic acid ester, and (ii) subjecting the compound V to a condensation reaction with the obtained reaction mixture in (i) in the presence of a metal catalyst. The compound III may be obtained by subjecting the compound V to a condensation reaction with the compound IV (Y¹ represents MgY³) in the presence of a metal catalyst.

When Y¹ represents MgY³, Y³ preferably represents chlorine or bromine.

When Y¹ represents ZnY³ or B(OR⁴)₂, the compound III can be obtained by (i) subjecting the compound V to a condensation reaction with the compound IV in the presence of a metal catalyst.

When Y¹ represents ZnY³ or B(OR⁴)₂, the compound IV (Y¹ represents ZnY³ or B(OR⁴)₂) may be synthesized from the compound IV (Y¹ represents MgY³), or a compound IV (Y¹ represents ZnY³ or B(OR⁴)₂) may be synthesized by a commonly known method separately. Here, Y³ in ZnY³ and Y³ in MgY³ may be the same as or different from each other.

When Y¹ represents ZnY³, Y³ preferably represents chlorine, bromine, or iodine.

An amount of the reagent used is not particularly limited, and for example, with respect to the compound V, the amount of the compound IV is preferably 1.0 to 2.0 equivalent, and more preferably 1.5 to 2.0 equivalent, the amount of the Grignard reagent is preferably 1.0 to 2.0 equivalent, and more preferably 1.5 to 2.0 equivalent, the amount of the zinc halide is preferably 1.0 to 3.0 equivalent, and more preferably 1.0 to 2.0 equivalent, the amount of the organodiboron or boronic acid ester is preferably 1.0 to 5.0 equivalent, and more preferably 1.5 to 4.0 equivalent, and the amount of the metal catalyst is preferably 0.05 to 0.20 equivalent, and more preferably 0.05 to 0.10 equivalent.

The compound IV is a commercially available product, or is available by synthetic method in accordance with a method described in a known document (for example, ORGANIC LETTERS, 2010, Vol.12, No.20, 4632-4635, or the like).

The Grignard reagent is not particularly limited, and examples thereof include isopropyl magnesium chloride, isopropyl magnesium bromide, isopropyl magnesium chloride-lithium chloride, and isopropyl magnesium bromide-lithium chloride. Isopropyl magnesium chloride is preferred.

The zinc halide is not particularly limited, and examples thereof include zinc chloride, zinc bromide, and zinc iodide. Zinc chloride is preferred.

The organodiboron is not particularly limited, and examples thereof include pinacol diboron, catechol diboron, neopentyl glycol diboron, and N-methyliminodiacetate diboron.

The boronic acid ester is not particularly limited, and examples thereof include a trimethoxyboronic acid ester and 2-isopropoxy-4,4,5,5-tetramethyl-1,3,2-dioxaborane.

When the obtained boronic acid ester derivative (Y¹ represents B(OR⁴)₂, and R⁴ is not H) is hydrolyzed, the boronic acid derivative (Y¹ represents B(OR⁴)₂, and R⁴ represents H. That is, Y¹ represents B(OH)₂) may be obtained.

The compound IV (Y¹ represents B(OR⁴)₂) may be isolated and then subjected to a condensation reaction with the compound V.

The compound IV is not particularly limited, and examples thereof include compounds represented by the structures shown below.

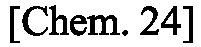

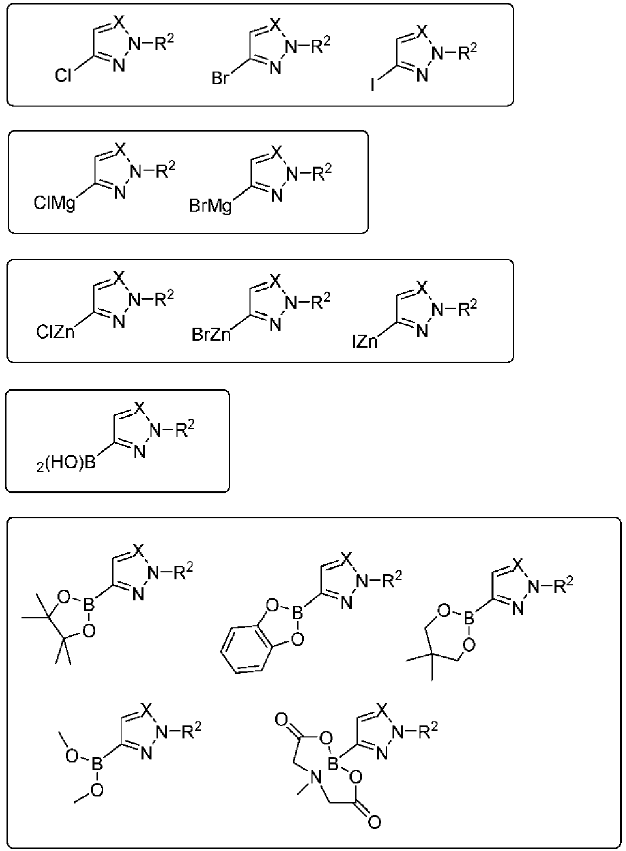

The metal catalyst is not particularly limited as long as it is a metal catalyst used in cross-coupling, and examples thereof include [1,1'-bis(diphenylphosphino)ferrocene]palladium(II) dichloride-dichloromethane adduct, [1,1'-bis(diphenylphosphino)ferrocene]palladium(II) dichloride, bis(triphenylphosphine)palladium(II) dichloride, [1,3-bis(2,6-di-3-pentylphenyl)imidazol-2-ylidene](3-chloropyridyl)palladium(II) dichloride, chloro[[1,3-bis(2,6-diisopropylphenyl)imidazol-2-ylidene](4'-methoxyacetanilide)palladium(II)], tris(dibenzylideneacetone)dipalladium, palladium acetate, tetrakis(triphenylphosphine)palladium, and nickel bromide diglyme salt. Preferred are [1,1'-bis(diphenylphosphino)ferrocene]palladium(II) dichloride-dichloromethane adduct and [1,1'-bis(diphenylphosphino)ferrocene]palladium(II) dichloride.

The solvent is not particularly limited, and examples thereof include THF, methyl tert-butyl ether, dioxane, toluene, dimethylformamide, and dimethylacetamide. These may be used alone or in combination. THF is preferred.

The amount of the solvent used is not particularly limited, and is, for example, 4 to 10 volume/weight (v/w) with respect to the compound IV in the stage of (i), 10 to 20 volume/weight (v/w) with respect to the compound IV in the stage of (ii), and 20 to 30 volume/weight (v/w) with respect to the compound V in the stage of (iii).

The reaction in the stage of (i) is preferably performed at a temperature between 0°C and 40°C and more preferably at a temperature between 10°C and 30°C, generally for 3 hours to 10 hours.

The reaction in the stage of (ii) is preferably performed at a temperature between 0°C and 40°C and more preferably at a temperature between 10°C and 20°C, generally for 30 minutes to 2 hours.

The reaction in the stage of (iii) is preferably performed at a temperature between 10°C and 50°C and more preferably at a temperature between 20°C and 30°C, generally for 1 hour to 3 hours.

By using activated carbon for post-treatment after the reaction in (iii), a metal species derived from a metal catalyst such as a palladium catalyst can be removed and decolored.

In step 3, (i) the Grignard reagent may be reacted with the compound V, next, (ii) the obtained reaction mixture may be reacted with zinc halide, organodiboron, or boronic acid ester, and (iii) the compound IV and the reaction mixture obtained in (i) or (ii) may be subjected to a condensation reaction in the presence of a metal catalyst. In this case, in the description of step 3, the compound V may be replaced with the compound IV, and the compound IV may be replaced with the compound V.

### Step 4

The compound I can be obtained by reacting the compound II and the compound III in an appropriate solvent in the presence of a base.

An amount of each reagent used is not particularly limited, but for example, the amount of the compound II is preferably 1.0 to 1.5 equivalent and more preferably 1.0 to 1.2 equivalent, and the amount of the base is preferably 1.0 to 3.0 equivalent, and more preferably 1.5 to 2.5 equivalent. with respect to the amount of the compound III.

The compound II is available by synthesis according to a method described in a known document (for example, WO2011/142316).

The base is not particularly limited, and examples thereof include inorganic salts such as cesium carbonate, sodium hydroxide, a mixture of sodium hydroxide and potassium carbonate, potassium hydroxide, sodium hydride, and potassium tert-butoxide. Cesium carbonate is preferred.

The solvent is not particularly limited, and examples thereof include toluene, chlorobenzene, THF, 2-MeTHF, acetonitrile, and ethyl acetate. These may be used alone or in combination. Toluene is preferred.

The amount of the solvent used is not particularly limited, and is, for example, 10 to 30 volume/weight (v/w) with respect to the compound III.

The reaction is generally performed at a temperature between 50°C and 110°C and more preferably at a temperature between 90°C and 110°C, generally for 3 hours to 15 hours.

Inorganic salts such as cesium salts, sodium salts, and potassium salts can be removed and decolored by using the activated carbon in the post-treatment after the reaction.

### Step 5

The compound IX can be obtained by a condensation reaction using the compound VII and the compound IV in the same manner as in step 3. Here, in the description of step 3, the compound V may be replaced with the compound VII.

### Step 6

The compound VIII can be obtained by a reaction for generating a trifluoroacetyl group from a carboxy ester group in the compound IX using a trifluoromethylating agent in the same manner as in step 1. Here, in the description of step 1, the compound VII may be replaced with the compound IX.

### Step 7

The compound VIII can be obtained by a condensation reaction using the compound VI and the compound IV in the same manner as in the step 3. Here, in the description of step 3, the compound V may be replaced with the compound VI.

### Step 8

The compound III can be obtained by the asymmetric reduction reaction of the trifluoroacetyl group in the compound VIII in the same manner as in step 2. Here, in the description of step 2, the compound VI may be replaced with the compound VIII.

Steps 5 to 8 may be performed by appropriately replacing the solvent, the reagent, the reaction condition, and the like described in steps 1 to 3 in accordance with a starting material and a reaction product.

A product and an intermediate in each of the above steps can be isolated and purified by separation and purification methods commonly used in organic synthetic chemistry, such as filtration, extraction, washing, drying, concentration, recrystallization, and various types of chromatography. The product or intermediate in each step may also be provided for the following reaction without being particularly purified.

In addition, a raw material compound, an obtained intermediate, and a product used in each step may be in the form of a salt or a solvate.

When it is desired to obtain salts of the intermediate and the product obtained in each step, the intermediate and the product obtained in each step may be purified as they are when they are obtained in the form of salts. When they are obtained in a free form, the intermediate and product obtained in each step may be dissolved or suspended in a suitable solvent, and an acid or base may be added thereto to form a salt, which may then be isolated and purified.

Examples of salts of the raw material compound used in each step and salts of the obtained intermediate and product include an acid addition salt, a metal salt, an ammonium salt, an organic amine addition salt, and an amino acid addition salt. Examples of the acid addition salts include inorganic acid salts such as hydrochlorides, hydrobromides, nitrates, sulfates, and phosphates, and organic acid salts such as acetates, oxalates, maleates, fumarates, citrates, benzoates, and methanesulfonates. Examples of the metal salts include alkali metal salts such as sodium salts and potassium salts, alkaline earth metal salts such as magnesium salts and calcium salts, aluminum salts, and zinc salts. Examples of the ammonium salts include ammonium and tetramethylammonium. Examples of the organic amine addition salts include addition salts of morpholine, piperidine, or the like. Examples of the amino acid addition salts include addition salts of lysine, glycine, phenylalanine, aspartic acid, glutamic acid, or the like.

When it is desired to obtain solvates of the raw material compound used in each step and the obtained intermediate and product, the solvates may also be obtained directly by the above production method or the like. Alternatively, the solvates may be obtained by mixing the raw material compound used in each step and the obtained intermediate or product with each solvent to form solvates and isolating and purifying the solvents.

When the compounds such as raw material compounds, intermediates, and products are in the form of salts, the salts of the compounds may be pharmacologically acceptable salts of the compounds. The above salts can be used as pharmacologically acceptable salts.

According to the method for producing the compound I based on the above scheme, the compound I can be easily obtained in a shorter process and at a higher yield than a known method (for example, Patent Literature 1). In addition, the production method is suitable as an industrial production method because the compound I can be efficiently produced with a certain quality and good reproducibility.

As described above, the compound I may be present not only as a free form but also as a salt or a solvate thereof, and may be present in a crystalline state. Crystals of the compound I, crystals of the salt thereof, or crystals of a solvate thereof may also have polymorphism or crystal habits. For example, the compound I includes a crystal of the compound I, a crystal of a salt of the compound I, a crystal of a solvate of the compound I, a crystal of a solvate of a salt of the compound I, various polymorphism of thereof and various crystal habits thereof. More specifically, examples of the compound IA include a type III crystal of the compound IA and a type II crystal of the compound IA, which are described in Examples described below. These crystalline forms can be identified by, for example, measuring powder X-ray diffraction, and the measurement values of powder X-ray diffraction described in the present description are measured by a transmission method. The measurement value (2θ) of the powder X-ray diffraction may vary in a range of ±0.2°.

The type II crystal of the compound IA may be directly obtained by the above method (step 4), and can be produced by, for example, the following method.

The compound IA is dissolved in toluene at a temperature between 40°C and 70°C, preferably at a temperature between 55°C and 65°C, then heptane is added dropwise while stirring, and the mixture is cooled to a temperature between 10°C and 30°C. Thus, the type II crystal of the compound IA can be obtained with good yield and reproducibility.

Toluene is used in an amount of, for example, 5 to 30 volume/weight (v/w), preferably 10 to 25 volume/weight (v/w), more preferably 10 to 15 volume/weight (v/w), with respect to the compound IA. In addition, heptane is used in an amount of, for example, 5 to 30 volume/weight (v/w), preferably 10 to 25 volume/weight (v/w), and more preferably 10 to 15 volume/weight (v/w), with respect to the compound IA.

The compound IA as a raw material is not particularly limited, and may be a compound obtained in the above step 4. It is preferable to use the compound IA with a high purity in order to maintain the quality of the compound IA as a pharmaceutical product. A seed crystal (type II crystal) produced separately may be added during cooling as necessary. The seed crystal (type II crystal) can be obtained by precipitation without using a seed crystal according to the present method.

The type III crystal of the compound IA can be produced by, for example, the following method.

The compound IA is dissolved in an alcohol-based solvent (for example, methanol) at a temperature between 20°C and 70°C, preferably at a temperature between 30°C and 65°C, then water is added dropwise while stirring, and the mixture is cooled to a temperature between 20°C and 30°C. Thus, a type III crystal of the compound IA can be obtained with good yield and reproducibility.

Here, the form of the compound IA used is not particularly limited, and examples of the form of the compound IA include a mixture containing the compound IA, an amorphous form of the compound IA, a type II crystal of the compound IA, and a type III crystal of the compound IA.

Examples of the alcohol-based solvent include lower alcohols such as methanol and ethanol, that is, aliphatic alcohols having 1 to 10 carbon atoms.

The alcohol-based solvent is used in an amount of, for example, 5 to 30 volume/weight (v/w), preferably volume/weight (v/w), and more preferably volume/weight (v/w), with respect to the compound IA. The water is used in an amount of, for example, 5 to 30 volume/weight (v/w), preferably 5 to 25 volume/weight (v/w), and more preferably 10 to 20 volume/weight (v/w), with respect to the compound IA.

Crystals that do not transition to other forms even under severe conditions such as high temperature and high humidity are excellent from the viewpoint of pharmaceutical production requiring stable supply.

As described above, the compound IA has a plurality of crystalline forms such as a type III crystal and a type II crystal, and among them, the type III crystal is excellent in stability (see Test Example 1 described below), can be stored for a long period of time with a certain quality, and is suitable as a drug for an active pharmaceutical ingredient.

The crystal of the compound IA or the crystal of the salt of the compound IA can be used as an active ingredient of a pharmaceutical preparation by being granulated by pulverization or the like as necessary, for example, as a therapeutic and/or preventive agent for a disease involved in kynurenine generation and/or the expression of indoleamine 2,3-dihydrogenase. Among them, the above crystals can be used as a treatment and/or preventive agent of, for example, cancer (tumor), immune diseases, neurodegenerative diseases, and infectious diseases.

The crystal of the compound IA or the crystal of the salt of the compound IA can be administered alone as it is, and it is generally preferable to provide them as various pharmaceutical preparations. The pharmaceutical preparation is used for animals or humans.

The pharmaceutical preparation contains, as an active ingredient, a crystal of the compound IA or a crystal of a salt of the compound IA alone or a mixture with an active ingredient for any other treatment.

The pharmaceutical preparation may be produced by mixing the active ingredient with one or more pharmaceutically acceptable carriers (for example, a diluent, a solvent, and an excipient) using any method well known in the technical field of pharmaceutical science. Examples of the pharmaceutical preparation include a kynurenine generation inhibitor and an indoleamine 2,3-dihydrogenase inhibitor. As another example of the pharmaceutical preparation, a treatment and/or preventive agent for a disease associated with kynurenine generation and/or indoleamine 2,3-dihydrogenase expression is exemplified.

As the route of administration, it is preferable to use the most effective route for treatment, and oral administration or parenteral administration such as intravenous administration can be exemplified.

The form of administration is not particularly limited, and examples thereof include tablets, external preparations, and injections.

For example, tablets suitable for oral administration can be produced using, for example, an excipient such as lactose, a disintegrant such as starch, a lubricant such as magnesium stearate, and a binder such as hydroxypropyl cellulose.

Examples of the external preparations which are suitable for parenteral administration include an ointment, a cream, a liniment, a lotion, a poultice, a plaster, and a tape. For example, an ointment, a cream, or the like can be produced by dissolving or mixing and dispersing an active ingredient in a basic agent such as white petrolatum.

For example, an injection suitable for parenteral administration can be produced using a diluent or a solvent such as a salt solution, a glucose solution, or a mixed solution of saline and a glucose solution.

The dose and the number of times of administration of a crystal of the compound IA or a crystal of a salt of the compound IA are different depending on the administration form, age and body weight of a patient, nature or severity of a symptom to be treated, and the like. In the case of oral administration, administration is performed once to several times per adult in the range of generally 0.01 to 1000 mg, preferably 0.05 to 100 mg. In the case of parenteral administration such as intravenous administration, administration is performed once to several times per adult in the range of generally 0.001 to 1000 mg, preferably 0.01 to 200 mg. The dose and the number of times of administration are not limited to the above ranges, and may vary under the various conditions described above.

The compound IA has an action of inhibiting generation of kynurenine, and thus has an action of inhibiting an increase in kynurenine (Patent Literature 1). Therefore, a crystal of the compound IA or a crystal of a salt of the compound IA also has an action of inhibiting the generation of kynurenine, and thus has an action of inhibiting the increase in kynurenine. Since the crystal of the compound IA or the crystal of the salt of the compound IA has an excellent action of inhibiting the production of kynurenine, the crystal of the compound IA or the crystal of the salt of the compound IA is useful for, for example, preventing or treating a disease involved in the generation of kynurenine and/or the expression of indoleamine 2,3-dihydrogenase.

The crystal of the compound IA or the crystal of the salt of the compound IA may be used as an indoleamine 2,3-dihydrogenase inhibitor.

Examples of diseases involved in kynurenine production and/or indoleamine 2,3-dihydrogenase expression include cancer (tumor), immune diseases, neurodegenerative diseases, and infectious diseases.

Examples of the cancer (tumor) include hematopoietic tumors, multiple myeloma, breast cancer, ovarian cancer, uterine cancer, cervical cancer, prostate cancer, bladder cancer, renal cancer, stomach cancer, esophageal cancer, liver cancer, cholangiocarcinoma, colon cancer, rectal cancer, pancreatic cancer, lung cancer, head and neck cancers, osteosarcoma, melanoma, and brain tumors. The crystal of the compound IA or the crystal of the salt of the compound IA is suitable for prevention or treatment of cancers such as stomach cancer or breast cancer.

Examples of the immune diseases include acquired immune deficiency syndrome (AIDS), bronchial asthma, pollinosis, allergic rhinitis, atopic dermatitis, rheumatoid arthritis, ulcerative colitis, Crohn's disease, multiple sclerosis, amyotrophic lateral sclerosis, and graft-versus-host disease.

Examples of neurodegenerative diseases include AIDS dementia, Alzheimer's disease, and depression.

Examples of the infectious diseases include viral infections, bacterial infections, fungal infections, chlamydial infections, and rickettsial infections.

### [Examples]

Hereinafter, the present invention will be described more specifically with reference to Examples and Reference Examples, but the scope of the present invention is not limited to these Examples.

The proton nuclear magnetic resonance spectroscopy (¹H-NMR) used in Examples and Reference Examples were measured at 300 MHz, 400 MHz, or 500 MHz, interchangeable protons may not be clearly observed depending on the compound and the measurement conditions. It should be noted that the multiplicity of signals is indicated by the usual notation, and br indicates an apparently broad signal.

In addition, the measurement of powder X-ray diffraction (reflection method) was performed by pulverizing a sample in an agate mortar, taking an appropriate amount of the sample into a sample plate, and measuring a diffraction peak such that a diffraction angle 2θ includes a range of 5° to 40°. The X-ray to be radiated was a copper Kα ray (CuKα ray), and the tube voltage was set to 50 kV, the tube current was set to 1 mA, and the step time was set to 120 seconds. The thermal analysis was performed by taking 2 mg of the sample into an aluminum pan and measuring differential scanning calorimetry (DSC) under a condition of a heating rate of 5°C/ min. For the measurement of powder X-ray diffraction (reflection method), D8 DISCOVER manufactured by Bruker AXS was used.

### [Example 1] Method for Producing Type III Crystal of Compound IA

### (Step 1)

Methyl 6-chloronicotinate (30.0 g, 0.175 mol) was dissolved in 2-MeTHF (270 mL) at room temperature in a nitrogen atmosphere. While stirring the mixture at room temperature, (trifluoromethyl)trimethylsilane (49.7 g, 0.350 mol) was added dropwise to the mixture, and 2-MeTHF (30 mL) was added to the mixture. Subsequently, a suspension of 2-ethylhexanoic acid (0.63 g, 4.37 mmol) and cesium carbonate (3.99 g, 12.2 mmol) in 2-MeTHF (3 mL) was added dropwise to the mixture at 0°C to 5°C, and 2-MeTHF (6 mL) was added. After the mixture was stirred at the same temperature for 0.5 hours, 6.0 mol/L hydrochloric acid (150 mL) was added dropwise to the mixture, and the mixture was stirred at room temperature for 24 hours. A 10 mol/L aqueous sodium hydroxide solution (about 90 mL) was added dropwise to the mixture until the pH of the mixture reached 6.5. Then, the thus-obtained liquid was allowed to stand until an interface became clear, and the thus-obtained liquid was separated into an organic layer and an aqueous layer. The 12 wt% saline (300 mL) was added dropwise to the obtained organic layer, followed by stirring at room temperature. Thereafter, the thus-obtained liquid was allowed to stand until an interface became clear, and the thus-obtained liquid was separated into an organic layer and an aqueous layer. The obtained organic layer was concentrated up to 120 mL or less under reduced pressure at 40°C or lower.

The residue was prepared to 270 mL using 2-MeTHF, and triethylamine (35 g, 0.35 mol) and formic acid (9.7 g, 0.21 mol) were added dropwise thereto while stirring at room temperature, and 2-MeTHF (24 mL) was added to the mixture. Subsequently, a THF (3 mL) solution of (pentamethylcyclopentadienyl)iridium (III) dichloride (dimer) (17 mg, 0.022 mmol) and (R,R)-N-(2-amino-1,2-diphenylethyl)-p-toluenesulfonamide (17 mg, 0.046 mmol) was added dropwise to the mixture at 30°C, and 2-MeTHF (6 mL) was added to the mixture. After stirring the mixture at the same temperature for 3 hours (optical purity 97.9% ee by HPLC of the reaction solution), water (300 mL) was added to the mixture. Thereafter, 6.0 mol/L hydrochloric acid (about 47 mL) was added dropwise until the pH of the mixture reached 6.5, followed by stirring at room temperature for 30 minutes. Then, the thus-obtained liquid was allowed to stand until an interface became clear, and then the thus-obtained liquid was separated into an organic layer and an aqueous layer. To the obtained organic layer, 2-MeTHF (30 mL) suspension of activated carbon (3 g) was added dropwise, and 2-MeTHF (15 mL) was added to the mixture, followed by stirring at room temperature for 1 hour. The mixture was filtered to remove insoluble matter, followed by washing with 2-MeTHF (60 mL). The filtrate was concentrated to 60 mL or less under reduced pressure at 40°C or lower, and toluene (300 mL) was added. The obtained concentrated residue was concentrated to 60 mL or less under reduced pressure at 40°C or lower, and toluene (300 mL) was added. The concentrated residue was concentrated again to 60 mL or less under reduced pressure at 40°C or lower, and toluene (300 mL) was added. The concentrated residue was concentrated under reduced pressure at 40°C or lower and the volume was adjusted to 120 mL with toluene. The solution was heated to 50°C, and dissolution was confirmed, followed by cooling to 35°C over 1 hour or longer. After crystal precipitation was confirmed, stirring was performed at the same temperature for 1 hour. A mixed solution of toluene (30 mL) and heptane (375 mL) was added to the suspension at 35°C to 40°C over 1 hour, followed by stirring at the same temperature for 1 hour. The suspension was cooled to 25°C over 1 hour and stirred at the same temperature for 12 hours. The precipitated solid was collected by filtration and washed with a mixed solution of toluene and heptane (mixing ratio: 1:9, 90 mL). The obtained solid was dried under reduced pressure at 30°C or lower to obtain (R)-1-(6-chloropyridin-3-yl)-2,2,2-trifluoroethanol (33.14 g, yield: 90%, optical purity: 100% ee).

¹H NMR (DMSO-d₆, δ, ppm): 5.4 (m, 1H), 7.19 (d, J = 3.2 Hz, 1H), 7.61 (d, J = 8.5 Hz, 1H), 7.97 (d of doublets, J = 2.4, 8.3 Hz, 1H), 8.52 (d, J = 2.4 Hz, 1H).

### (Step 2)

In a nitrogen atmosphere, 4-bromo-2-methyl-2H-1,2,3-triazole (21.4 g, 0.132 mmol) obtained by a method known in the literature (ORGANIC LETTERS, 2010, Vol. 12, No. 20, 4632-4635) was dissolved in THF (60 mL) at room temperature. The mixture was cooled to 0°C to 5°C, and a THF solution (86 mL, 0.17 mmol) of 2.0 mol/L isopropyl magnesium chloride was added dropwise at an internal temperature of 20°C or lower, followed by stirring at 15°C for 3 hours. A suspension of zinc chloride (15.5 g, 0.113 mmol) in THF (172 mL) was added dropwise to the obtained mixture at an internal temperature of 20°C or lower, followed by stirring at 15°C for 1 hour. The mixture was heated to 25°C, [1,1'-bis(diphenylphosphino)ferrocene]palladium(II) dichloride-dichloromethane adduct (3.86 g, 4.73 mmol) was added to the mixture, and THF (80 mL) was added. At the same temperature, (R)-1-(6-chloropyridin-3-yl)-2,2,2-trifluoroethanol (20.0 g, 94.5 mmol) obtained in step 1 was added dropwise to the mixture, and THF (200 mL) was added, followed by stirring for 1 hour. An aqueous solution (300 mL) obtained by dissolving ammonium acetate (109 g, 1.42 mol) in 300 mL of water was added to the mixture, followed by stirring at 15°C for 20 minutes. The thus-obtained liquid was allowed to stand at the same temperature until an interface became clear, and the thus-obtained liquid was separated into an organic layer and an aqueous layer. To the obtained organic layer was added 2 mol/L hydrochloric acid (20 mL), followed by stirring at 15°C for 10 minutes, activated carbon (2.0 g) was added, and the mixture was stirred at 15°C for 15 hours. The mixture was filtered to remove insoluble matter, followed by washing with THF (60 mL). An aqueous solution (300 mL) obtained by dissolving ammonium chloride (76 g, 1.42 mol) in 300 mL of water was added to the filtrate, followed by stirring at 20°C for 30 minutes. The thus-obtained liquid was allowed to stand at the same temperature until an interface became clear, and the thus-obtained liquid was separated into an organic layer and an aqueous layer. The obtained organic layer was concentrated up to 100 mL or less under reduced pressure at 45°C or lower, toluene (300 mL) was added to the concentrated residue, and the resultant was concentrated up to 100 mL or less under reduced pressure. Toluene (300 mL) was added to the concentrated residue, the resultant was concentrated up to 100 mL or less under reduced pressure, and toluene was added thereto to adjust the volume to 200 mL. The obtained mixture was stirred at 20°C, water (100 mL) was added thereto, and 6 mol/L hydrochloric acid (100 mL) was added, followed by stirring at 5°C for 1 hour. The mixture was filtered to remove insoluble matter, followed by washing with 0.5 mol/L hydrochloric acid (60 mL). The filtrate was stirred at 20°C for 10 minutes, allowed to stand at the same temperature until an interface became clear, and the thus-obtained liquid was separated into an organic layer and an aqueous layer. A suspension of activated carbon (2.0 g) in water (20 mL) was added to the obtained aqueous layer, followed by stirring at 20°C for 1 hour. The mixture was filtered to remove insoluble matter, followed by washing with 0.5 mol/L hydrochloric acid (60 mL). The filtrate was stirred at 15°C, methanol (20 mL) was added, and a 4 mol/L aqueous sodium hydroxide solution (120 mL) was added dropwise at 20°C. The temperature was increased to 37°C, a 4 mol/L aqueous sodium hydroxide solution (about 23 mL) was added to adjust the pH to 1.5, and (R)-2,2,2-trifluoro-1-[6-(2-methyl-2H-1,2,3-triazol-3-yl)pyridin-3-yl]ethanol (20 mg) was added as seed crystals to confirm crystallization. Then, stirring was performed at the same temperature for 1 hour. A 4 mol/L aqueous sodium hydroxide solution (about 20 mL) was added to the mixture to adjust the pH to 3.5, followed by stirring at the same temperature for 1 hour. After slowly cooling to 32°C, stirring was performed at the same temperature for 2 hours. The precipitated solid was collected by filtration and washed with water (60 mL). The obtained solid was dried under reduced pressure at 40°C or lower to obtain (R)-2,2,2-trifluoro-1-[6-(2-methyl-2H-1,2,3-triazol-3-yl)pyridin-3-yl]ethanol (20.3 g, yield: 90%, optical purity: 100% ee). In this step, crystals of the target substance may be precipitated without adding seed crystals. In the above production method, crystals obtained by natural growth were added as seed crystals.

¹H NMR (DMSO-d₆, δ, ppm): 4.25 (s, 3H), 5.4 (m, 1H), 7.11 (d, J = 5.7 Hz, 1H), 7.97 (d of doublets, J = 0.7, 8.2 Hz, 1H), 8.01 (d of doublets, J = 2.0, 8.2 Hz, 1H), 8.26 (s, 1H), 8.72 (d, J = 1.6 Hz, 1H).

### (Step 3)

In a nitrogen atmosphere, toluene (75 mL) was added to (R)-2,2,2-trifluoro-1-[6-(2-methyl-2H-1,2,3-triazol-3-yl)pyridin-3-yl]ethanol (5.0 g, 19 mmol) obtained in step 2, N-(3-chloroquinoxalin-2-yl)propane-1-sulfonamide (5.8 g, 20 mmol) obtained by a method known in the literature (WO2011/142316), and cesium carbonate (12.6 g, 38.7 mmol) at room temperature, and the suspension was stirred. The temperature was increased to 105°C, and the mixture was stirred for 2 hours. The mixture was cooled to 45°C or lower, 0.4 mol/L hydrochloric acid (about 60 mL) was added, and the pH was adjusted to 7.5. The mixture was stirred at 35°C for 1 hour and confirmed to have a pH of 7.5. The mixture was allowed to stand at the same temperature until an interface became clear, and the mixture was separated into an organic layer and an aqueous layer. To the obtained organic layer was added 3 wt% saline (75 mL) at 35°C, followed by stirring, and 0.4 mol/L hydrochloric acid (about 1 mL) was added, and the pH was adjusted to 7.0. The mixture was stirred for 30 minutes and confirmed to have a pH of 7.0 at 35°C. The mixture was allowed to stand at the same temperature until an interface became clear, and the mixture was separated into an organic layer and an aqueous layer. A suspension of activated carbon (0.5 g) in toluene (5 mL) was added to the obtained organic layer to, and the mixture was followed by stirring at 30°C for 1 hour. The mixture was filtered to remove insoluble matter, followed by washing with toluene (15 mL). The filtrate was concentrated up to 60 mL or less under reduced pressure at 60°C or lower, and the volume was adjusted to 60 mL with toluene. The concentrated residue was heated to 60°C, and dissolution was confirmed. Then, heptane (50 mL) was added dropwise at the same temperature over 30 minutes or longer. As a seed crystal, (R)-N-(3-{2,2,2-trifluoro-1-[6-(2-methyl-2H-1,2,3-triazol-4-yl)pyridin-3-yl]ethoxy}quinoxalin-2-yl)propane-1-sulfonamide (10 mg) was added to confirm crystallization. Then, stirring was performed at the same temperature for 1 hour or longer. Heptane (90 mL) was added dropwise at the same temperature over 3 hours, followed by stirring at the same temperature for 1 hour or longer. Cooling was performed to an internal temperature of 25°C over 3 hours, and stirring was performed at the same temperature for 3 hours. The precipitated solid was collected by filtration and washed with a mixed solution of toluene and heptane (mixing ratio: 1:2, 45 mL). The obtained solid was dried under reduced pressure at 60°C or lower to obtain a type II crystal (8.8 g, yield: 90%, optical purity: 100% ee) of (R)-N-(3-{2,2,2-trifluoro-1-[6-(2-methyl-2H-1,2,3-triazol-4-yl)pyridin-3-yl]ethoxy}quinoxalin-2-yl)propane-1-sulfonamide (compound IA). In this step, crystals of the target substance may be precipitated without adding seed crystals. In the above production method, crystals obtained by natural growth were added as seed crystals.

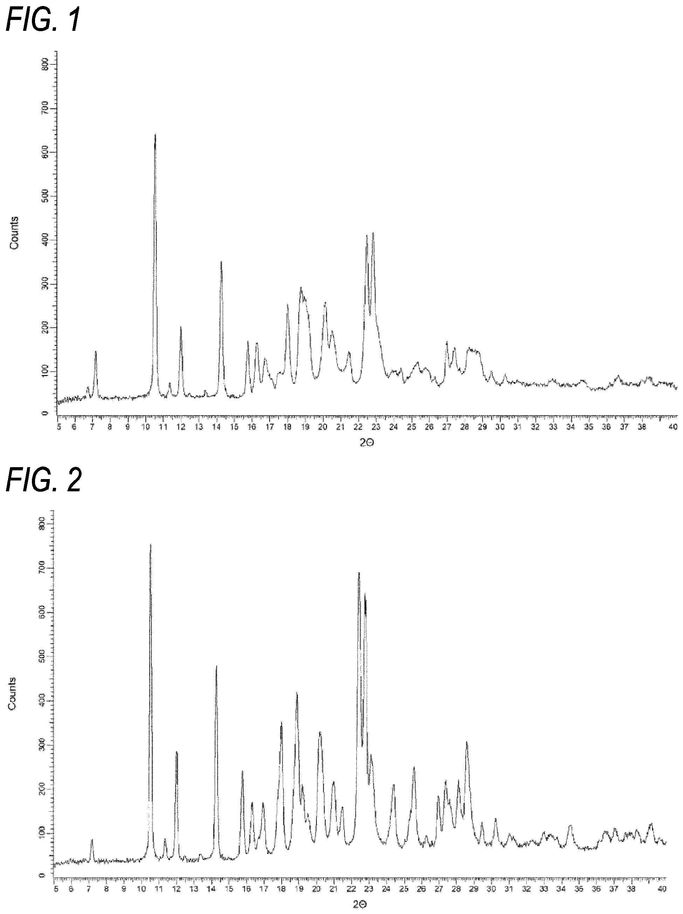

Powder X-ray diffraction measurement results: main peaks were shown at diffraction angles (2θ) = 10.5°, 12.0°, 14.3°, 15.8°, 16.3°, 16.7°, 18.0°, 20.1°, 20.5°, 22.5°, and 22.8° (see FIG. 1).

Thermal analysis (DSC) measurement results: an endothermic peak was shown at about 137°C.

### (Step 4)

In a nitrogen atmosphere, the type II crystal (8.0 g, 15.8 mmol) of the compound IA obtained in step 3 was suspended in methanol (80 mL) at room temperature. The mixture was heated to 35°C, and dissolution was confirmed. Then, a suspension of activated carbon (0.8 g) in methanol (8 mL) was added to the mixture, followed by stirring at the same temperature for 1 hour. The mixture was filtered to remove insoluble matter, followed by washing with methanol (32 mL). The filtrate was heated to 60°C, water (40 mL) was added, and dissolution was confirmed. After cooling to 50°C over 15 minutes, crystallization was confirmrf. Then, stirring was performed at the same temperature for 1 hour. The mixture was cooled to 40°C over 30 minutes and stirred at the same temperature for 1 hour. Water (40 mL) was added dropwise to the mixture over 1 hour, and the mixture was stirred at the same temperature for 1 hour. The internal temperature was increased again to 60°C over 30 minutes, and the mixture was stirred at the same temperature for 15 hours or longer. The mixture was cooled to 25°C over 1 hour and stirred at the same temperature for 3 hours. The precipitated solid was collected by filtration and washed with a mixed solution of methanol and water (mixing ratio: 6:4, 40 mL). The obtained solid was dried under reduced pressure at 60°C or lower to obtain a type III crystal of the compound IA (7.5 g, yield: 94%, optical purity: 100% ee).

¹H NMR (DMSO-d₆, δ, ppm): 1.07 (t, J = 7.5 Hz, 3H), 1.90 (d of sextet, J = 3.0, 7.5 Hz, 2H), 3.8 (m, 2H), 4.28 (s, 3H), 7.3 (m, 1H), 7.6 (m, 2H), 7.78 (d, J = 6.3 Hz, 1H), 7.88 (d, J = 8.3 Hz, 1H), 8.07 (d, J = 8.0 Hz, 1H), 8.31 (s, 1H), 8.51 (d, J =8.0 Hz, 1H), 9.20 (s, 1H), 11.47 (s, 1H).

Powder X-ray diffraction measurement results: main peaks were shown at diffraction angles (2θ) = 10.5°, 12.0°, 14.3°, 15.8°, 16.3°, 17.0°, 18.0°, 18.9°, 20.2°, 21.0°, 22.4°, 22.8°, 23.1°, 24.4°, and 25.6° (see FIG. 2).

Thermal analysis (DSC) measurement results: an endothermic peak was shown at about 139°C.

### [Example 2A] Alternative Method 1 of Steps 1 to 2 in Example 1

### (Step 1)

Methyl 6-chloronicotinate (10 g, 0.058 mol) was dissolved in 2-MeTHF (100 mL) at room temperature in a nitrogen atmosphere. While stirring at room temperature, cesium fluoride (0.9 g, 5.8 mmol) was added to the mixture. Subsequently, (trifluoromethyl)trimethylsilane (16.6 g, 0.12 mol) was added dropwise to the mixture at 0°C to 5°C. After the mixture was stirred at the same temperature for 4 hours, 6.0 mol/L hydrochloric acid (50 mL) was added dropwise to the mixture, and the mixture was stirred at room temperature for 19 hours. After adding 2-MeTHF (80 mL) to the mixture, 10 mol/L aqueous sodium hydroxide solution (about 16 mL) was added dropwise until the pH of the mixture reached 6.6. Thereafter, the thus-obtained liquid was allowed to stand until an interface became clear, and the thus-obtained liquid was separated into an organic layer and an aqueous layer. The 10 wt% saline (100 mL) was added dropwise to the obtained organic layer, followed by stirring at room temperature. Thereafter, the thus-obtained liquid was allowed to stand until an interface became clear, and the thus-obtained liquid was separated into an organic layer and an aqueous layer. Magnesium sulfate (4 g) was added to the obtained organic layer, followed by stirring at room temperature. The mixture was filtered to remove insoluble matter, followed by washing with 2-MeTHF (10 mL), to obtain a 2-MeTHF solution of 1-(6-chloropyridin-3-yl)-2,2,2-trifluoroethane-1-one.

### (Step 2)

A half amount of the 2-MeTHF solution of 1-(6-chloropyridin-3-yl)-2,2,2-trifluoroethane-1-one obtained in step 1 was used to prepare 200 mL of solution with 2-MeTHF. While stirring at room temperature, [(R,R)-N-(2-amino-1,2-diphenylethyl)-p-toluenesulfonamide]chloro(mesitylene)ruthenium(II) (91 mg, 0.15 mmol) and formic acid (2.0 g, 0.044 mol) were added dropwise. Subsequently, the mixture was cooled to 0°C, triethylamine (3.2 g, 0.032 mol) was added to the mixture, and the mixture was stirred at 20°C for 16 hours (optical purity: 92.0% ee according to HPLC of the reaction solution), followed by concentrating the mixture. Ethyl acetate (100 mL) and water (50 mL) were added to the concentrated residue, and 3.0 mol/L hydrochloric acid (about 0.4 mL) was added dropwise thereto until the pH of the mixture reached 6.3. After stirring at room temperature, the mixture was allowed to stand and separated into an organic layer and an aqueous layer. To the obtained organic layer was added 12 wt % saline (50 mL) while stirring. After stirring the mixture at room temperature, the mixture was allowed to stand and separated into an organic layer and an aqueous layer. A suspension of activated carbon (1 g) and magnesium sulfate (1 g) in ethyl acetate (10 mL) was added dropwise to the obtained organic layer, and the mixture was stirred at room temperature for 1 hour. The mixture was filtered to remove insoluble matter, followed by washing with ethyl acetate (10 mL). The filtrate was concentrated to 50 mL or less under reduced pressure at 40°C or lower and was prepared to 50 mL with ethyl acetate. A hydrochloric acid (4.0 mol/L) ethyl acetate solution (about 14 mL) was added dropwise at room temperature, and the precipitation of crystals was confirmed, followed by stirring at the same temperature for 19 hours. The precipitated solid was collected by filtration and washed with a mixed solution of ethyl acetate and heptane (mixing ratio: 1:1, 10 mL). The obtained solid was dried under reduced pressure at 30°C or lower to obtain (R)-1-(6-chloropyridin-3-yl)-2,2,2-trifluoroethanol hydrochloride (5.73 g, 2-step total yield: 79%, optical purity: 96.8% ee).

¹H NMR (CDCl3, δ, ppm): 5.1 (m, 1H), 7.45 (d, J = 7.8 Hz, 1H), 7.88 (d, J = 8.1 Hz, 1H), 8.52 (s, 1H).

### (Step 3)

In an argon atmosphere, 4-bromo-2-methyl-2H-1,2,3-triazole (13.5 g, 83.2 mmol) obtained by a method known in the literature (ORGANIC LETTERS, 2010, Vol. 12, No. 20, 4632-4635) was dissolved in THF (34 mL) at room temperature. The mixture was cooled to 0°C to 5°C, and a THF solution (54.1 mL, 108 mmol) of 2.0 mol/L isopropyl magnesium chloride was added dropwise at an internal temperature of 15°C or lower, followed by stirring at 3°C for 4 hours. A suspension of zinc chloride (14.7 g, 108 mmol) in THF (108 mL) was added dropwise to the obtained mixture at an internal temperature of 25°C or lower, followed by stirring at 16°C for 1 hour. The obtained mixture is referred to as MTZ solution. The (R)-1-(6-chloropyridin-3-yl)-2,2,2-trifluoroethanol hydrochloride (15.0 g, 60.48 mmol) obtained in step 2 was dissolved in a mixed solvent of methyl tert-butyl ether (105 mL) and water (22.5 mL). While stirring at room temperature, a 4 mol/L aqueous sodium hydroxide solution (about 15 mL) was added dropwise until the pH of the mixture reached 7.5. After stirring at room temperature, the thus-obtained liquid was allowed to stand to separate into an organic layer and an aqueous layer. A saline (200 mL) was added to the obtained organic layer, and the mixture was stirred at room temperature, allowed to stand, and separated into an organic layer and an aqueous layer. The obtained organic layer was concentrated up to 30 mL or less under reduced pressure. THF (75 mL) was added to the obtained residue, the thus-obtained solution was concentrated up to 30 mL or less under reduced pressure, and 189 mL of a solution was prepared with THF. Using 159 mL of the obtained solution, [1,1'-bis(diphenylphosphino)ferrocene]palladium(II) dichloride-dichloromethane adduct (2.43 g, 2.97 mmol) was added at room temperature to obtain a mixed solution. The MTZ solution previously obtained was added dropwise to the mixed solution at 40°C or lower, followed by stirring at room temperature for 3.5 hours. Water (12.6 mL) was added to the mixture, followed by concentrating up to 164 mL under reduced pressure. Toluene (126 mL, 10 v/w) was added to the concentrated residue, and 0.5 mol/L hydrochloric acid (113 mL) was added at room temperature. While stirring at room temperature, 12 mol/L hydrochloric acid (1 mL) was added dropwise until the pH of the mixture reached 3.5. After stirring at room temperature, the thus-obtained liquid was allowed to stand to separate into an organic layer and an aqueous layer. Water (37 mL) and 6 mol/L hydrochloric acid (88 mL) were added to the obtained organic layer, followed by stirring for 1 hour under ice cooling. The precipitated solid was collected by filtration and washed with 0.5 mol/L hydrochloric acid (37 mL). The obtained filtrate was stirred at room temperature and then allowed to stand to separate into an organic layer and an aqueous layer. Toluene (126 mL) was added to the obtained aqueous layer, and a 10 mol/L aqueous sodium hydroxide solution (about 50 mL) was added dropwise at an internal temperature of 30°C or lower until the pH of the mixture reached 3.5. After stirring at room temperature, the mixture was allowed to stand to separate into an organic layer and an aqueous layer, and a 12 wt% aqueous sodium hydroxide solution (38 mL) and activated carbon (2.52 g) were added to the organic layer, followed by stirring at room temperature for 1 hour. The activated carbon was filtrated, followed by washing with ethyl acetate (400 mL). The filtrate was allowed to stand at room temperature to separate into an organic layer and an aqueous layer. The obtained organic layer was concentrated up to 63 mL under reduced pressure, and (R)-2,2,2-trifluoro-1-(6-(2-methyl-2H-1,2,3-triazol-3-yl)pyridin-3-yl)ethanol (3.2 mg) obtained in step 2 of Example 1 was added as a seed crystal at an internal temperature of 30°C to the concentrated residue, followed by stirring at room temperature overnight. The mixture was heated to 35°C, and n-heptane (101 mL) was added dropwise over about 6 hours. After cooling to an internal temperature of 15°C and stirring for 1 hour or longer, the precipitated solid was collected by filtration and washed with a mixed solvent of toluene and n-heptane (mixing ratio: 1:3, 37 mL). The obtained solid was dried under reduced pressure at 50°C or lower to obtain (R)-2,2,2-trifluoro-1-[6-(2-methyl-2H-1,2,3-triazol-3-yl)pyridin-3-yl]ethanol (12.9 g, yield: 84%, optical purity: 99.6% ee).

### [Example 2B] Alternative Method 2 of Steps 1 and 2 of Example 1

### (Step 1)

A half amount of the 2-MeTHF solution of 1-(6-chloropyridin-3-yl)-2,2,2-trifluoroethane-1-one obtained in step 1 of Example 2A was concentrated to obtain 8.1 g of concentrated residue. The concentrated residue (3.8 g) was purified by column chromatography to obtain 1-(6-chloropyridin-3-yl)-2,2,2-trifluoroethane-1-one (1.1 g, yield: 40%).

¹H NMR (CDCl3, δ, ppm): 7.56 (d, J = 7.8 Hz, 1H), 8.29 (d, J = 8.1 Hz, 1H), 9.05 (s, 1H).

### (Step 2)

In a nitrogen atmosphere, 1-(6-chloropyridin-3-yl)-2,2,2-trifluoroethane-1-one (200 mg, 0.95 mmol) obtained in step 1 was dissolved in 2-MeTHF (4 mL) at room temperature. While stirring at room temperature, formic acid (54 µL, 1.4 mmol), (pentamethylcyclopentadienyl)iridium (III) dichloride (dimer) (7.6 mg, 0.0095 mmol), and (R,R)-N-(2-amino-1,2-diphenylethyl)-p-toluenesulfonamide (14 mg, 0.038 mmol) were added. Subsequently, the mixture was cooled to 0°C, and triethylamine (146 µL, 1.0 mmol) was added, followed by stirring at the same temperature for 2 hours. As a result of HPLC analysis of the obtained solution, a peak having a retention time matching that of (R)-1-(6-chloropyridin-3-yl)-2,2,2-trifluoroethanol obtained in step 1 of Example 1 was 99.0 area% (99.7% ee).

### (Step 3)

Using (R)-1-(6-chloropyridin-3-yl)-2,2,2-trifluoroethanol obtained in step 2, (R)-2,2,2-trifluoro-1-[6-(2-methyl-2H-1,2,3-triazol-3-yl)pyridin-3-yl]ethanol is obtained in the same manner as in step 2 of Example 1.

### [Example 2C] Alternative Method 3 of Steps 1 and 2 of Example 1

### (Step 1)

In a nitrogen atmosphere, 1-(6-chloropyridin-3-yl)-2,2,2-trifluoroethane-1-one (200 mg, 0.95 mmol) obtained in step 1 of Example 2B was dissolved in 2-MeTHF (4 mL) at room temperature. While stirring at room temperature, formic acid (54 µL, 1.4 mmol), (pentamethylcyclopentadienyl)rhodium(III) dichloride (dimer) (5.8 mg, 0.0094 mmol), and (R,R)-N-(2-amino-1,2-diphenylethyl)-p-toluenesulfonamide (14 mg, 0.038 mmol) were added. Subsequently, the mixture was cooled to 0°C, and triethylamine (146 µL, 1.0 mmol) was added, followed by stirring at the same temperature for 2 hours. As a result of HPLC analysis of the obtained solution, a peak having a retention time matching that of (R)-1-(6-chloropyridin-3-yl)-2,2,2-trifluoroethanol obtained in step 1 of Example 1 was 98.4 area% (99.6% ee).

### (Step 2)

Using (R)-1-(6-chloropyridin-3-yl)-2,2,2-trifluoroethanol obtained in step 1, (R)-2,2,2-trifluoro-1-[6-(2-methyl-2H-1,2,3-triazol-3-yl)pyridin-3-yl]ethanol is obtained in the same manner as in step 2 of Example 1.

### [Example 2D] Alternative Method 4 of Steps 1 and 2 of Example 1

### (Step 1)

In a nitrogen atmosphere, 1-(6-chloropyridin-3-yl)-2,2,2-trifluoroethane-1-one (200 mg, 0.95 mmol) obtained in step 1 of Example 2B was dissolved in THF (2 mL) at room temperature. The mixture was cooled to 0°C, and sodium formate (195 mg, 2.9 mmol) and [N-[(1R,2R)-2-(amino-κN)-1,2-diphenylethyl]-4-methylbenzenesulfonamide-κN]chloro[(1,2,3,4,5-η)-1,2,3,4,5-pentamethyl-2,4-cyclopentadiene-1-yl]iridium (7.1 mg, 0.0095 mmol) obtained by a method known in the literature (Chemistry Letters, 1998, 1199-1200) were added thereto, followed by stirring at the same temperature for 1 hour, and then stirring is performed at 30°C for 3 hours. As a result of HPLC analysis of the obtained solution, a peak having a retention time matching that of (R)-1-(6-chloropyridin-3-yl)-2,2,2-trifluoroethanol obtained in step 1 of Example 1 was 98.9 area% (98.7% ee).

### (Step 2)

Using (R)-1-(6-chloropyridin-3-yl)-2,2,2-trifluoroethanol obtained in step 1, (R)-2,2,2-trifluoro-1-[6-(2-methyl-2H-1,2,3-triazol-3-yl)pyridin-3-yl]ethanol is obtained in the same manner as in step 2 of Example 1.

### [Example 2E] Alternative Method 5 of Steps 1 and 2 of Example 1

### (Step 1)

In a nitrogen atmosphere, 1-(6-chloropyridin-3-yl)-2,2,2-trifluoroethane-1-one (200 mg, 0.95 mmol) obtained in step 1 of Example 2B was dissolved in 2-propanol (4 mL) at room temperature. While stirring at room temperature, (pentamethylcyclopentadienyl)iridium (III) dichloride (dimer) (7.6 mg, 0.0095 mmol) and (R,R)-N-(2-amino-1,2-diphenylethyl)-p-toluenesulfonamide (14 mg, 0.038 mmol) were added. Subsequently, the mixture was cooled to 0°C, and diazabicycloundecene (157 µL, 1.0 mmol) was added thereto, followed by stirring at room temperature for 4 hours in a hydrogen atmosphere. As a result of HPLC analysis of the obtained solution, a peak having a retention time matching that of (R)-1-(6-chloropyridin-3-yl)-2,2,2-trifluoroethanol obtained in step 1 of Example 1 was 97.0 area% (98.4% ee).

### (Step 2)

Using (R)-1-(6-chloropyridin-3-yl)-2,2,2-trifluoroethanol obtained in step 1, (R)-2,2,2-trifluoro-1-[6-(2-methyl-2H-1,2,3-triazol-3-yl)pyridin-3-yl]ethanol is obtained in the same manner as in step 2 of Example 1.

### [Example 2F] Alternative Method 6 of Steps 1 and 2 of Example 1

### (Step 1)

In a nitrogen atmosphere, a coenzyme present in Chiralscreen (registered trademark) OH screening kit (catalog number: 01005) sold from Daicel Corporation was dissolved in water (6 mL) at room temperature. To the NADH-dependent enzyme E031 of the same kit was added 1 mL of the obtained solution. Thereafter, 1-(6-chloropyridin-3-yl)-2,2,2-trifluoroethane-1-one (5 mg, 0.024 mmol) obtained in step 1 of Example 2B was added, followed by stirring at 25°C for 14 hours. As a result of HPLC analysis of the obtained solution, a peak having a retention time matching that of (R)-1-(6-chloropyridin-3-yl)-2,2,2-trifluoroethanol obtained in step 1 of Example 1 was 99.7 area% (99.7% ee).

### (Step 2)

Using (R)-1-(6-chloropyridin-3-yl)-2,2,2-trifluoroethanol obtained in step 1, (R)-2,2,2-trifluoro-1-[6-(2-methyl-2H-1,2,3-triazol-3-yl)pyridin-3-yl]ethanol is obtained in the same manner as in step 2 of Example 1.

### [Example 3] Alternative Method 7 of Steps 1 and 2 of Example 1

### (Step 1)

After 4-bromo-2-methyl-2H-1,2,3-triazole (0.39 mol/L THF solution) (2.4 mL, 0.93 mmol) was ice-cooled, isopropyl magnesium chloride-lithium chloride (1.3 mol/L THF solution) (2.8 mL, 3.7 mmol) was added to the solution, followed by stirring for 1 hour under ice cooling. Zinc chloride (250 mg, 1.9 mmol) was added to the mixed solution, followed by stirring at room temperature for 2 hours. Methyl 6-bromonicotinate (50 mg, 0.23 mmol) and [1,1'-bis(diphenylphosphino)ferrocene]palladium(II) dichloride-dichloromethane adduct (19 mg, 0.023 mmol) were added to the mixed solution, followed by stirring at room temperature for 2 hours. After the mixed solution was diluted to 50 mL, it was confirmed that 82% of methyl 6-(2-methyl-2H-1,2,3-triazole-4-yl)nicotinate was generated in this step by HPLC quantification, using methyl 6-(2-methyl-2H-1,2,3-triazole-4-yl)nicotinate obtained in step 5 of Reference Example 1 as a standard product.

### (Step 2)

THF (10 mL), (trifluoromethyl)trimethylsilane (0.51 mL, 3.4 mmol), and cesium fluoride (35 mg, 0.23 mmol) were added to methyl 6-(2-methyl-2H-1,2,3-triazole-4-yl)nicotinate (500 mg, 2.3 mmol) obtained in step 1, followed by stirring at 30°C for 1 hour. To the mixed solution were added (trifluoromethyl)trimethylsilane (0.17 mL, 1.2 mmol) and cesium fluoride (18 mg, 0.12 mmol), followed by stirring at 30°C for 0.5 hours. A 6 mol/L aqueous hydrochloric acid solution (10 mL) and THF (5 mL) were added to the obtained mixed solution, followed by stirring for 1.5 hours and then standing overnight, and a mixed solution was obtained. Ethyl acetate (10 mL) was added to the mixed solution, and then pH was adjusted to 7.4 with a 6 mol/L aqueous sodium hydroxide solution, followed by liquid separation. After extracting the aqueous layer with ethyl acetate (10 mL), all the organic layers were merged, and a saturated saline solution (10 mL) was added to separate the liquid. The obtained organic layer was dried over magnesium sulfate and then concentrated under reduced pressure. A mixed solution of ethyl acetate and heptane (mixing ratio: 2:8) (5 mL) was added to the concentrated residue, and the mixture was stirred at 50°C for 1 hour, and then was cooled to room temperature and allowed to stand overnight. After standing overnight, the mixture was stirred at room temperature for 0.5 hours, and the precipitated solid was collected by filtration and dried under reduced pressure at 50°C to obtain 2,2,2-trifluoro-1-[6-(2-methyl-2H-1,2,3-triazol-4-yl)pyridin-3-yl]ethanone (450 mg, yield: 76%).

¹H NMR (CDCl3, δ, ppm): 4.31 (s, 3H), 8.09 (d, J = 8.7 Hz, 1H), 8.26 (s, 1H), 8.39 (d, J = 8.1 Hz, 1H), 9.28 (s, 1H).

### (Step 3A)

In a nitrogen atmosphere, 2,2,2-trifluoro-1-[6-(2-methyl-2H-1,2,3-triazol-4-yl)pyridin-3-yl]ethanone (200 mg, 0.78 mmol) obtained in step 2 was dissolved in 2-MeTHF (4 mL) at room temperature. While stirring at room temperature, formic acid (66 µL, 1.2 mmol), (pentamethylcyclopentadienyl)iridium (III) dichloride (dimer) (7.6 mg, 0.0095 mmol), and [(R,R)-N-(2-amino-1,2-diphenylethyl)-p-toluenesulfonamide] (14 mg, 0.038 mmol) were added to the mixture. Subsequently, the mixture was cooled to 0°C, and triethylamine (179 µL, 0.86 mmol) was added, followed by stirring at the same temperature for 2 hours. As a result of HPLC analysis of the obtained solution, a peak having a retention time matching that of (R)-2,2,2-trifluoro-1-(6-(2-methyl-2H-1,2,3-triazol-3-yl)pyridin-3-yl)ethanol obtained in step 2 of Example 1 was 98.9 area% (97.9% ee).

### (Step 3B)

In a nitrogen atmosphere, 2,2,2-trifluoro-1-[6-(2-methyl-2H-1,2,3-triazol-4-yl)pyridin-3-yl]ethanone (200 mg, 0.78 mmol) obtained in step 2 was dissolved in 2-MeTHF (4 mL) at room temperature. While stirring at room temperature, formic acid (66 µL, 1.2 mmol), (pentamethylcyclopentadienyl)rhodium(III) dichloride (dimer) (5.8 mg, 0.0094 mmol), and [(R,R)-N-(2-amino-1,2-diphenylethyl)-p-toluenesulfonamide] (14 mg, 0.038 mmol) were added. Subsequently, the mixture was cooled to 0°C, and triethylamine (179 µL, 0.86 mmol) was added, followed by stirring at the same temperature for 2 hours. As a result of HPLC analysis of the obtained solution, a peak having a retention time matching that of (R)-2,2,2-trifluoro-1-[6-(2-methyl-2H-1,2,3-triazol-3-yl)pyridin-3-yl]ethanol obtained in step 2 of Example 1 was 98.0 area% (96.7% ee).

### (Step 3C)

In a nitrogen atmosphere, a coenzyme present in Chiralscreen (registered trademark) OH screening kit sold from Daicel Corporation was dissolved in 6 mL of water at room temperature to obtain a coenzyme solution, and 1 mL of the coenzyme solution was charged into a container containing NADH-dependent enzyme E031. Thereafter, 2,2,2-trifluoro-1-[6-(2-methyl-2H-1,2,3-triazol-4-yl)pyridin-3-yl]ethanone (5 mg, 0.020 mmol) obtained in step 2 was added, followed by stirring at 25°C for 14 hours. As a result of HPLC analysis of the obtained solution, a peak having a retention time matching that of (R)-2,2,2-trifluoro-1-[6-(2-methyl-2H-1,2,3-triazol-3-yl)pyridin-3-yl]ethanol obtained in step 2 of Example 1 was 99.7 area% (99.8% ee).

### [Example 4] Alternative Method 8 of Steps 1 and 2 of Example 1

### (Step 1)

THF (10 mL), (trifluoromethyl)trimethylsilane (0.86 mL, 5.8 mmol), and cesium fluoride (70 mg, 0.46 mmol) were added to methyl 6-bromonicotinate (1.0 g, 4.6 mmol), and a mixed solution was followed by stirring at room temperature for 0.5 hours. To the mixed solution were added (trifluoromethyl)trimethylsilane (0.17 mL, 1.2 mmol) and cesium fluoride (70 mg, 0.46 mmol), followed by stirring at room temperature for 0.5 hours. A 6 mol/L aqueous hydrochloric acid solution (10 mL) was added to the obtained mixed solution, followed by stirring for 0.5 hours and then standing overnight. THF (10 mL) was added to the mixed solution, followed by stirring at room temperature for 0.5 hours, ethyl acetate (20 mL) was added thereto, pH was adjusted to 7.5 with a 6 mol/L aqueous sodium hydroxide solution, and liquid separation was performed. A mixed solution (15 mL) obtained by diluting a saturated saline solution 1.5 times with water was added to the organic layer to perform liquid separation. The obtained organic layer was dried over magnesium sulfate and then concentrated under reduced pressure. The residue was purified by column chromatography to obtain 1-(6-bromopyridin-3-yl)-2,2,2-trifluoroethane-1-one (800 mg, yield: 68%).

Under the NMR measurement conditions of ¹H NMR (CDCl3, δ, ppm), a mixture of 1-(6-bromopyridin-3-yl)-2,2,2-trifluoroethane-1-one (ketone form) and 1-(6-bromopyridin-3-yl)-2,2,2-trifluoroethane-1,1-diol (diol form) was observed.

¹H NMR (CDCl3, δ, ppm) of 1-(6-bromopyridin-3-yl)-2,2,2-trifluoroethane-1-one: 7.73 (d, J = 8.4 Hz, 1H), 8.15 - 8.19 (m, 1H), 9.00 - 9.01 (m, 1H).

¹H NMR (CDCl3, δ, ppm) of 1-(6-bromopyridin-3-yl)-2,2,2-trifluoroethane-1,1-diol: 4.46 (s, br, 2H), 7.54 (d, J = 8.4 Hz, 1H), 7.89 (dd, J = 8.3, 2.6 Hz, 1H), 8.62 (d, J = 2.7 Hz, 1H).

### (Step 2)

After a THF solution of 4-bromo-2-methyl-2H-1,2,3-triazole (670 mg, 4.2 mmol) was ice-cooled, isopropyl magnesium chloride-lithium chloride (1.3 mol/L THF solution) (4.8 mL, 6.2 mmol) was added, followed by stirring for 0.5 hours under ice cooling. To the mixed solution was added isopropyl magnesium chloride-lithium chloride (1.3 mol/L THF solution) (1.6 mL, 2.1 mmol), followed by stirring for 1 hour under ice cooling. To the mixed solution was added 2-isopropoxy-4,4,5,5-tetramethyl-1,3,2-dioxaborane (1.3 mL, 6.2 mmol), followed by stirring for 1 hour under ice cooling. To the mixed solution was added 2-isopropoxy-4,4,5,5-tetramethyl-1,3,2-dioxaborane (1.9 mL, 9.3 mmol), followed by stirring for 0.5 hours under ice cooling. To the mixed solution were added water (5 mL) and toluene (10 mL), and then, the pH of the mixed solution was adjusted to 7.5 with a 6 mol/L aqueous hydrochloric acid solution. The mixed solution was separated, and then a saturated saline solution (3 mL) was added to the organic layer to perform liquid separation. The obtained organic layer was dried over magnesium sulfate, concentrated under reduced pressure, and allowed to stand overnight. After adding heptane (20 mL) to the concentrate, the mixture was stirred at 0°C for 2 hours, and the precipitated crystals were filtered off. The obtained filtered liquid was concentrated under reduced pressure to obtain a crude product (1.6 g) of 2-methyl-4-(4,4,5,5-tetramethyl-1,3,2-dioxaborane-2-yl)-2H-1,2,3-triazole.

### (Step 3)

To 1-(6-bromopyridin-3-yl)-2,2,2-trifluoroethane-1-one (50 mg, 0.20 mmol) obtained in step 1 were added [1,1'-bis(diphenylphosphino)ferrocene]palladium(II) dichloride-dichloromethane adduct (16 mg, 0.020 mmol) and potassium phosphate (100 mg, 0.049 mmol). A dioxane (0.50 mL) solution of a crude product (160 mg, 0.79 mmol) of 2-methyl-4-(4,4,5,5-tetramethyl-1,3,2-dioxaborane-2-yl)-2H-1,2,3-triazole obtained in step 2 was added to this mixture, and then the mixed solution was stirred at 80°C for 4 hours and allowed to stand overnight. After the mixed solution was diluted to 50 mL, it was confirmed that 87% of 2,2,2-trifluoro-1-[6-(2-methyl-2H-1,2,3-triazol-4-yl)pyridin-3-yl]ethanone was generated in this step by HPLC quantification, using 2,2,2-trifluoro-1-[6-(2-methyl-2H-1,2,3-triazol-4-yl)pyridin-3-yl]ethanone obtained in step 2 of Example 3 as a standard product.

### (Step 4)

Using 2,2,2-trifluoro-1-[6-(2-methyl-2H-1,2,3-triazol-4-yl)pyridin-3-yl]ethanone obtained in step 3, (R)-2,2,2-trifluoro-1-[6-(2-methyl-2H-1,2,3-triazol-3-yl)pyridin-3-yl]ethanol is obtained in the same manner as in step 3A of Example 3.

### [Reference Example 1] Synthesis method of standard product used in step 1 of Example 3

### (Step 1)

{4-[5-(diethoxymethyl)pyridin-2-yl]-1H-1,2,3-triazol-1-yl}methyl pivalate (70 g, 0.19 mol) obtained by a method known in the literature (WO2011/142316) and acetonitrile (700 mL) were mixed, and a 6 mol/L aqueous hydrochloric acid solution (23 mL) and water (140 mL) were added dropwise at room temperature. The mixed solution was stirred at 30°C for 2 hours, then adjusted to have a pH of 7.0 with a 6 mol/L aqueous sodium hydroxide solution, and ethyl acetate (700 mL) was added for liquid separation. The obtained organic layer was dried over magnesium sulfate and then filtered. Activated carbon (70 g) was added to the filtered liquid, followed by stirring at room temperature for 1 hour and filtering, and the thus-obtained filtered liquid was concentrated to 70 mL. Heptane (70 mL) was added dropwise to the concentrated liquid, and the mixture was stirred at room temperature for 1 hour, then cooled to 5°C, and stirred at the same temperature for 1 hour. The precipitated crystal was collected by filtration, washed with heptane (70 mL), and then dried at 60°C under reduced pressure to obtain [4-(5-formylpyridin-2-yl)-1H-1,2,3-triazol-1-yl]methyl pivalate (45 g, yield: 81%).

¹H NMR (CDCl3, δ, ppm): 1.21 (s, 9H), 6.33 (s, 2H), 8.27 (dd, J = 8.1, 2.1 Hz, 1H), 8.36 (d, J = 8.2 Hz, 1H), 8.51 (s, 1H), 9.06 (d, J = 2.0 Hz, 1H), 10.12 (s, 1H).

### (Step 2)

[4-(5-formylpyridin-2-yl)-1H-1,2,3-triazol-1-yl]methyl pivalate (45 g, 0.16 mol) obtained in step 1, dichloromethane (135 mL), water (45 mL), 2,2,6,6-tetramethylpiperidine 1-oxyl (4.9 g, 31 mmol) and bisacetoxyiodobenzene (50 g, 0.16 mol) were mixed, followed by stirring at 40°C for 1 hour. To the mixed solution were added 2,2,6,6-tetramethylpiperidine 1-oxyl (2.5 g, 16 mmol) and bisacetoxyiodobenzene (25 g, 78 mmol), followed by stirring at 40°C for 1 hour. The mixed solution was cooled to room temperature, stirred for 1 hour, then cooled to 5°C, and stirred at the same temperature for 1 hour. The precipitated crystals were collected by filtration, washed with dichloromethane (70 mL), and then dried at 50°C under reduced pressure to obtain 6-[1-(pivaloyloxymethyl)-1H-1,2,3-triazol-4-yl]nicotinic acid (44 g, yield: 93%).

¹H NMR (DMSO-d_{6,}δ, ppm): 1.13 (s, 9H), 6.40 (s, 2H), 8.17 (d, J = 8.2 Hz, 1H), 8.37 (dd, J = 8.1, 2.1 Hz, 1H), 8.83 (s, 1H), 9.09 (d, J = 2.1 Hz).

### (Step 3)

6-[1-(pivaloyloxymethyl)-1H-1,2,3-triazol-4-yl]nicotinic acid (44 g, 140 mmol) obtained in step 2, toluene (132 mL), and trimethylsilyldiazomethane (2 mol/L diethyl ether solution) (72 mL, 140 mmol) were mixed and heated to 30°C. Methanol (22 mL) was added dropwise to the mixed solution, followed by stirring at 30°C for 0.5 hours. Trimethylsilyldiazomethane (2 mol/L diethyl ether solution) (15 mL, 28 mmol) and methanol (22 mL) were added dropwise to the mixed solution, followed by stirring at 30°C for 15 minutes and further stirring at 50°C for 15 minutes. After the mixed solution was cooled to 30°C, heptane (440 mL) was added dropwise to the mixed solution, followed by stirring at 15°C for 1.5 hours. The precipitated crystals were collected by filtration, washed with heptane (80 mL), and then dried under reduced pressure to obtain a crude product of methyl 6-[1-(pivaloyloxymethyl)-1H-1,2,3-triazole-4-yl]nicotinate (38 g). Toluene (150 mL), heptane (150 mL), and methanol (38 mL) were added to the crude product of methyl 6-[1-(pivaloyloxymethyl)-1H-1,2,3-triazole-4-yl]nicotinate (38 g), followed by stirring at 50°C for 0.5 hours. Methanol (38 mL) was added dropwise to the mixed solution, followed by stirring at 35°C for 3 hours. The precipitated crystals were collected by filtration, and the obtained crystals were dried under reduced pressure at 50°C to obtain methyl 6-[1-(pivaloyloxymethyl)-1H-1,2,3-triazole-4-yl]nicotinate (17 g, yield: 37%).

¹H NMR (CDCl3, δ, ppm): 1.21 (s, 9H), 3.97 (s, 3H), 6.32 (s, 2H), 8.26 (dd, J = 8.2, 0.7 Hz, 1H), 8.39 (dd, J = 8.2, 2.1 Hz, 1H), 8.47 (s, 1H), 9.20 (dd, J = 2.1, 0.7 Hz, 1H).

### (Step 4)

Methyl 6-[1-(pivaloyloxymethyl)-1H-1,2,3-triazole-4-yl]nicotinate (10 g, 31 mmol) obtained in step 3 and methanol (100 mL) were mixed and heated to 50°C. Sodium methoxide (5.1 g, 94 mmol) was added to the mixed solution, followed by stirring at 50°C for 1 hour. After the mixed solution was cooled to 30°C, a mixed solution of methanol and sulfuric acid (mixing ratio = 4:1) was added dropwise to adjust the pH to 7.0, and then a 6 mol/L aqueous hydrochloric acid solution (150 mL) was added. The mixed solution was heated to 50°C, stirred for 4 hours, then cooled to room temperature, and allowed to stand overnight. A 6 mol/L aqueous sodium hydroxide solution was added to the mixed solution to adjust the pH to 6.5, followed by stirring at room temperature for 1 hour. The suspension was stirred at 5°C for 3 hours, the precipitated crystals were collected by filtration and washed with water (10 mL), and then the obtained crystals were dried at 60°C under reduced pressure to obtain a crude product of methyl 6-(1H-1,2,3-triazol-4-yl)nicotinate (8 g).

Ethyl acetate (80 mL) was added to the obtained crude product of methyl 6-(1H-1,2,3-triazol-4-yl)nicotinate (8 g), followed by stirring at 50°C for 1 hour and further stirring at 30°C for 1 hour. The precipitated crystals were collected by filtration and washed with ethyl acetate (40 mL), and then, the obtained crystals were dried at 50°C under reduced pressure to obtain methyl 6-(1H-1,2,3-triazol-4-yl)nicotinate (6.1 g, yield: 95%).

¹H NMR (DMSO-d₆, δ, ppm): 3.90 (s, 3H), 8.14 (d, J = 8.4 Hz, 1H), 8.38 (d, J = 8.4 Hz, 1H), 8.53 (s, 1H), 9.10 (s, 1H).

### (Step 5)

Diazabicycloundecene (25 mL) and ethyl acetate (25 mL) were added to methyl 6-(1H-1,2,3-triazol-4-yl)nicotinate (5 g, 25 mmol) obtained in step 4, followed by cooling to 0°C. Methyl p-toluenesulfonate (17 mL, 110 mmol) was added to the mixed solution, followed by stirring at room temperature for 2.5 hours. Ethyl acetate (15 mL), water (40 mL), and a 6 mol/L aqueous HCl solution were added to the mixed solution to adjust the pH to 6.5, acetonitrile (20 mL) was added, followed by heating to 45°C, and liquid separation was performed. The aqueous layer was extracted twice with ethyl acetate (25 mL), and all the organic layers were mixed. Then, a saturated saline solution (25 mL) was added to perform liquid separation. The obtained organic layer was concentrated under reduced pressure. Acetonitrile (15 mL) and water (15 mL) were added to the concentrated residue, and the thus-obtained liquid was heated to 50°C to slurry for 1 hour. The suspension was cooled to room temperature and stirred for 1 hour, and then acetonitrile (7 mL) was added. The mixture was heated to 50°C and was stirred for 1 hour, then cooled to room temperature and stirred for 1.5 hours. The crystals were filtered under reduced pressure and then dried at 60°C under reduced pressure.

Ethyl acetate (6 mL) and heptane (34 mL) were added to the obtained crystals, and then the mixed solution was heated to 50°C and stirred for 1 hour, and then cooled to 5°C and stirred for 1.5 hours. The precipitated crystals were collected by filtration and washed with a mixed solution of ethyl acetate and heptane (mixing ratio = 3:17) (10 mL), and then the obtained crystals were dried at 60°C under reduced pressure to obtain methyl 6-(2-methyl-2H-1,2,3-triazole-4-yl)nicotinate (2 g, yield: 37%).

¹H NMR (DMSO-d₆, δ, ppm): 3.91 (s, 3H), 4.26 (s, 3H), 8.05 (d, J = 8.3 Hz, 1H), 8.34 (s, 1H), 8.34 (dd, J = 8.3, 2.2 Hz, 1H), 9.11 (d, J = 2.2 Hz, 1H).

### [Test Example 1] Test for confirming that type III crystal of compound represented by formula (IA) is more stable than type II crystal of compound represented by formula (IA)

The type II crystal of (R)-N-(3-{2,2,2-trifluoro-1-[6-(2-methyl-2H-1,2,3-triazol-4-yl)pyridin-3-yl]ethoxy}quinoxalin-2-yl)propane-1-sulfonamide obtained in step 3 of Example 1 was allowed to stand for 8 weeks at room temperature. When the powder X-ray diffraction measurement was performed again, the transition to a mixed crystal of the type III crystal and the type II crystal obtained in step 4 of Example 1 was confirmed.

It should be noted that when the stability of the type III crystal of the compound represented by the formula (IA) at 25°C and 60% RH for 5 years was evaluated, there were no changes in the properties, related substances, optical isomers, content, or powder X-ray diffraction pattern, and the type III crystal of the compound represented by the formula (IA) was stable.

### INDUSTRIAL APPLICABILITY

The present invention can provide a method for producing a compound represented by the formula (I) and a crystal of a compound represented by the formula (IA). The production method according to the present invention is useful as an industrial production method for an active pharmaceutical ingredient. Further, the crystal of the compound represented by the formula (IA) of the present invention is useful as the active pharmaceutical ingredient.

## Claims

1. A method for producing a compound represented by the formula (I), the method comprising:
a step of reacting a compound represented by the formula (II) and a compound represented by the formula (III), 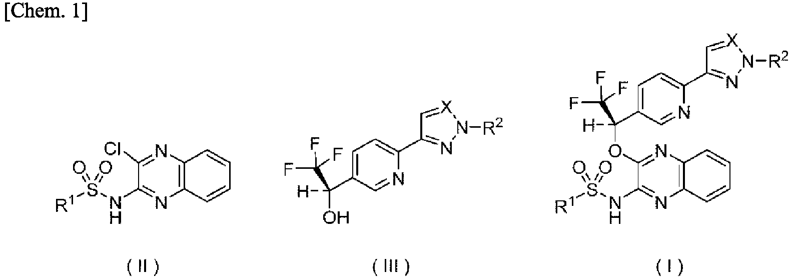
wherein R¹ represents lower alkyl which may be substituted with lower alkoxy or cycloalkyl, R² represents lower alkyl, and X represents N or CH.

2. The method according to claim 1, further comprising:
a step of obtaining the compound represented by the formula (III) from a compound represented by the formula (VI),
wherein the step is achieved by the following two reactions:
an asymmetric reduction reaction of a trifluoroacetyl group; and
a condensation reaction using a compound represented by the formula (IV), 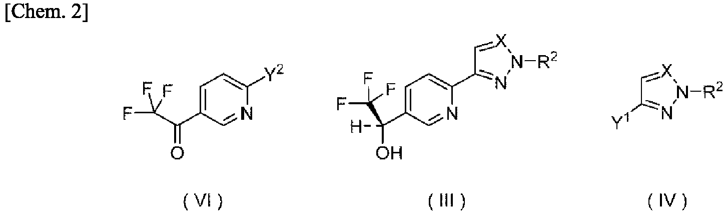
wherein R² represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other and represent H or lower alkyl, or form a ring structure together with boron.

3. The method according to claim 2, further comprising:
a step of subjecting the compound represented by the formula (VI) to an asymmetric reduction to obtain a compound represented by the formula (V); and
a step of reacting the compound represented by the formula (V) and the compound represented by the formula (IV) to obtain the compound represented by the formula (III), 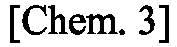 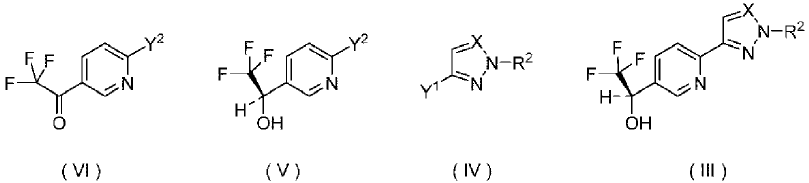
wherein R² represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other and represent H or lower alkyl, or form a ring structure together with boron.

4. The method according to claim 2, further comprising:
a step of reacting the compound represented by the formula (VI) and the compound represented by the formula (IV) to obtain a compound represented by the formula (VIII); and
a step of subjecting the compound represented by the formula (VIII) to asymmetric reduction to obtain the compound represented by the formula (III), 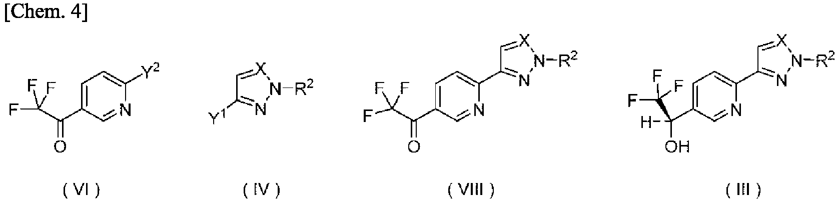
wherein R² represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other and represent H or lower alkyl, or form a ring structure together with boron.

5. The method according to claim 1, further comprising:
a step of obtaining the compound represented by the formula (III) from a compound represented by the formula (VII),
wherein the step is achieved by the following three reactions:
a reaction of generating a trifluoroacetyl group from a carboxy ester group using a trifluoromethylating agent;
an asymmetric reduction reaction of a trifluoroacetyl group; and
a condensation reaction using the compound represented by the formula (IV), 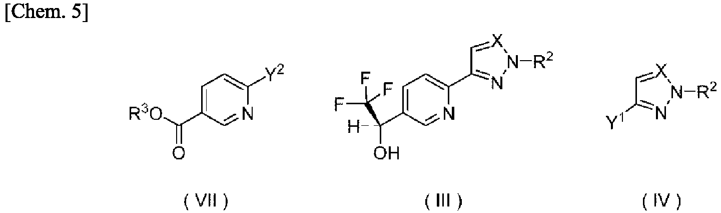
wherein R² represents lower alkyl, R³ represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other, and represent H or lower alkyl, or form a ring structure together with boron.

6. The method according to any one of claims 2 to 4, further comprising:
a step of reacting the compound represented by the formula (VII) and a trifluoromethylating agent to obtain the compound represented by the formula (VI), 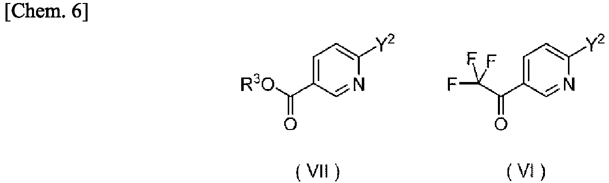
wherein R³ represents lower alkyl, and Y² represents halogen.

7. The method according to claim 1, further comprising:
a step of reacting the compound represented by the formula (VII) and the compound represented by the formula (IV) to obtain a compound represented by the formula (IX);
a step of reacting the compound represented by the formula (IX) and the trifluoromethylating agent to obtain the compound represented by the formula (VIII); and
a step of subjecting the compound represented by the formula (VIII) to asymmetric reduction to obtain the compound represented by the formula (III), 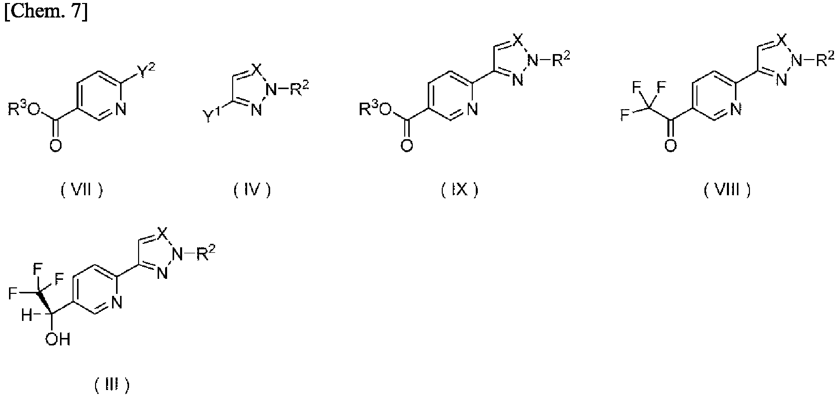
wherein R² represents lower alkyl, R³ represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other, and represent H or lower alkyl, or form a ring structure together with boron.

8. The method according to claim 1, further comprising:
a step of reacting the compound represented by the formula (V) and the compound represented by the formula (IV) to obtain the compound represented by the formula (III), 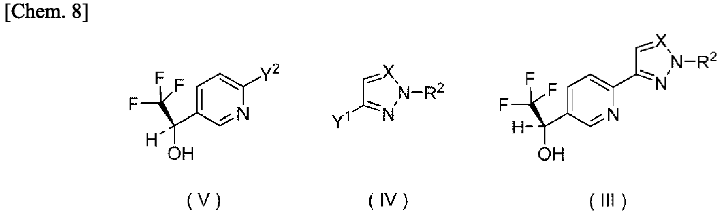
wherein R² represents lower alkyl, X represents N or CH, Y¹ represents halogen, M'Y³, or B(OR⁴)₂, Y² represents halogen, Y³ represents halogen, M' represents a metal species selected from zinc and magnesium, and R⁴s are the same as or different from each other and represent H or lower alkyl, or form a ring structure together with boron.

9. The method according to claim 1, further comprising:
a step of subjecting the compound represented by the formula (VIII) to asymmetric reduction to obtain the compound represented by the formula (III), 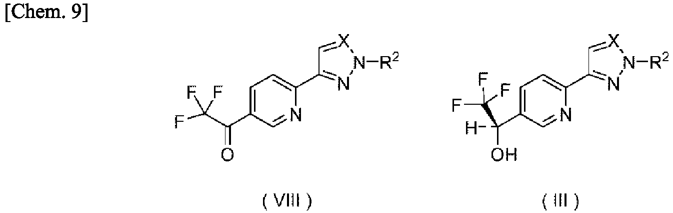
wherein R² represents lower alkyl, and X represents N or CH.

10. The method according to any one of claims 2 to 7 and 9, wherein an asymmetric reduction catalyst represented by the formula (M) is used in the asymmetric reduction reaction, 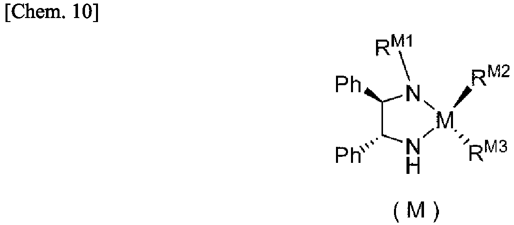 wherein M represents a metal species selected from ruthenium, rhodium, and iridium, R^{M1} represents p-toluene sulfonyl, methane sulfonyl, or benzyl sulfonyl, R^{M2} represents mesitylene, cumene, or pentamethylcyclopentadienyl, and R^{M3} is not present or represents chloro- or trifluoromethanesulfonate.

11. The method according to claim 10, wherein the asymmetric reduction catalyst represented by the formula (M) is formed in a reaction of (pentamethylcyclopentadienyl)iridium (III) dichloride (dimer) and [(R,R)-N-(2-amino-1,2-diphenylethyl)-p-toluenesulfonamide].

12. The method according to any one of claims 1 to 11, wherein R¹ represents propyl, R² represents methyl, and X represents N.

13. The method according to claim 12, further comprising: a step of recrystallizing the compound represented by the formula (I) using an alcohol-based solvent and water, 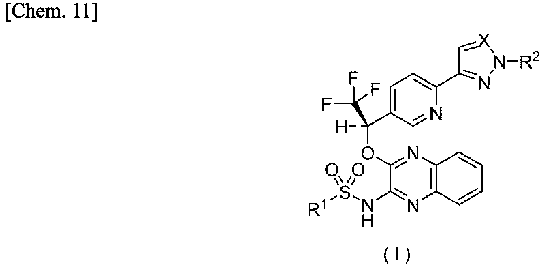 wherein R¹ represents lower alkyl which may be substituted with lower alkoxy or cycloalkyl, R² represents lower alkyl, and X represents N or CH.

14. A crystal of a compound represented by the formula (IA), having peaks at diffraction angles (2θ ± 0.2°) of 23.1°, 24.4°, and 25.6° in powder X-ray diffraction.
